# NEW EUROPEAN PATENT SPECIFICATION

(11) **EP 1 032 587 B2**
(45) Date of publication and mention of the opposition decision: **13.03.2013**
(45) Mention of the grant of the patent: 16.01.2008
(21) Application number: 98958573.2
(22) Date of filing: 13.11.1998
(51) Int. Cl.: A61K 38/16, C07K 14/46, C07K 14/575

(54) **NOVEL EXENDIN AGONIST COMPOUNDS**
NEUARTIGE EXENDIN AGONISTEN
NOUVEAUX COMPOSES AGONISTES DE L'EXENDINE

(30) Priority: 14.11.1997 US 66029 P
(43) Date of publication of application: 06.09.2000
(60) Divisional application: 07020413.6
(73) Proprietor: AMYLIN PHARMACEUTICALS, INC., San Diego, CA 92121 (US)
(72) Inventor: BEELEY, Nigel, Robert, Arnold, Solana Beach, CA 92131 (US); PRICKETT, Kathryn, S., San Diego, CA 92126 (US)
(74) Representative: HOFFMANN EITLE
(86) International application number: PCT/US1998/024273
(87) International publication number: WO 1999/025728

(56) References cited:
- EP-B1- 0 915 910
- EP-B1- 1 066 314
- WO-A-98/05351
- WO-A-98/30231
- WO-A-99/07404
- WO-A1-97/46584
- WO-A1-98/05351
- WO-A1-98/30231
- WO-A1-99/07404
- US-A- 694 954
- US-A- 5 424 286
- US-A- 5 424 286
- US-A- 5 424 286
- R. EISSELE ET AL.: 'RAT GASTRIC SOMATOSTATIN AND GASTRIN RELEASE:INTERACTIONS OF EXENDIN-4 AND TRUNCATED GLUCAGON-LIKE PEPTIDE-1 (GLP-1) AMIDE' LIFE SCIENCES vol. 55, no. 8, 1994, pages 629 - 634
- JOHN ENG ET AL.: 'Isolation and Characterization of Exendin-4,an Exendin-3 Analogue, from Heloderma suspectum Venum' THE JOURNAL OF BIOLOGICAL CHEMISTRY vol. 267, no. 11, 1992, pages 7402 - 7405
- RÜDIGER GÖKE ET AL.: 'Exendin-4 Is a High Potency Agonist and Truncated Exendin-(9-39)-amide an Antagonist at the Glucagon-like Peptide 1-(7-36)-amide Receptor of Insulin-secreting B-Cells' THE JOURNAL OF BIOLOGICAL CHEMISTRY vol. 268, no. 26, 1993, pages 19650 - 19655
- MARK GUTNAK ET AL.: 'ANTIDIABETOGENIC EFFECT OF GLUCAGON-LIKE PEPTIDE-1 (7-36)AMIDE IN NORMAL SUBJECTS AND PATIENTS WITH DIABETES MELLITUS' THE NEW ENGLAND JOURNAL OF MEDICINE vol. 326, no. 20, 1992, page 1316
- A G HARRIS: 'Somatostatin and somatostatin analogues: pharmacokinetics and pharmacodynamic effects' GUT vol. 35(3 SUPPL), 1994, pages 1 - 4
- J. N. HUNT ET AL.: 'Effect of Gastrin II on Gastric Emptying and Secretion During a Test Meal' BRIT.MED.J vol. 4, 1967, page 386-7
- R.L.LAWLER ET AL.: 'COMPARISON OF EFFECTS OF AMLYN, GLUCAGON-LIKE PEPTIDE-1 (GLP-1) AND EXENDIN-4 TO INHIBIT PENTAGASTRIN-STIMULATED GASTRIC ACID SECRETION IN RATS' GASTROENTEROLOGY vol. 112, no. 4, 1997, page A194
- M.D. TURTON ET AL.: 'A role for glucagon-like peptide-1 in the central regulation of feeding' NATURE vol. 379, no. 4, January 1996, pages 69 - 72
- ANDRÉ WETTERGREN ET AL.: 'Truncated GLP-1 (Proglucagon 78-107-Amide) Inhibits Gastric and Pancreatic Functions in Man' DIGESTIVE DISEASES AND SCIENCES vol. 38, no. 4, April 1993, pages 665 - 673
- ANA I.ALCANTARA ET AL.: 'Exendin-4 Agonist and Exendin(9-39)amide Antagonist of the GLP-1(7-36)amide Effects in Liver and Muscle' ARCHIVES OF BIOCHEMISTRY AND BIOPHYSICS vol. 341, no. 1, 01 May 1997, pages 1 - 7
- AMLYN: Grounds of Opposition against EP 0915910
- YIHONG WANG ET AL.: 'Glucagon-like Peptide-1 Can Reverse the Age-related Decline in Glucose Tolerance in Rats' THE JOURNAL OF CLINICAL INVESTIGATION vol. 99, no. 12, June 1997, pages 2883 - 2889
- STRAWMAN: Grounds of Opposition against EP 1066314B
- AMYLIN: Tables 1, 2, and 3 discussed herein
- AVI ASHKENAZI ET AL.: 'Mapping the CD4 binding site for human immunodeficiency virus by alanine-scanning mutagenesis' PROC. NATL. ACAD. SCI. USA vol. 87, September 1990, pages 7150 - 7154
- BRIAN C. CUNNINGHAM ET AL.: 'High-Resolution Epitope Mapping of hGH-Receptor Interactions by Alanine-Scanning Mutagenesis' SCIENCE vol. 244, 02 June 1989, pages 1081 - 1085
- KIM ADELHORST ET AL.: 'Structure-Activity Studies of Glucagon-like Peptide-1' THE JOURNAL OF BIOLOGICAL CHEMISTRY vol. 269, ISSUE OF MARCH 4, no. 9, 1994, pages 6275 - 6278
- CRAIG S. GIBBS ET AL.: 'Rational Scanning Mutagenesis of a Protein Kinase Identifies Functional Regions Involved in Catalysis and Substrate Interactions' THE JOURNAL OF BIOLOGICAL CHEMISTRY vol. 266, ISSUE OF MAY 15, no. 14, 1991, pages 8923 - 8931
- Data submitted by Patentee on 22.06.2010

### SEQUENCE LISTING

<110> AMYLIN PHARMACEUTICALS, INC.
<120> NOVEL EXENDIN AGONIST COMPOUNDS
<130> 238/087-EPO
<140> 98958573.2
   <141> 1998-11-13
<150> PCT/US98/24273
   <151> 1998-11-13
<150> US 60/066,029
   <151> 1997-11-14
<160> 110
<170> FastSEQ for Windows Version 3.0
<210> 1
   <211> 39
   <212> PRT
   <213> Heloderma horridum
<220>
   <221> AMIDATION
   <222> (39)...(39)
   <223> amidated Ser (Serinamide)
<400> 1
   His Ser Asp Gly Thr Phe Thr Ser Asp Leu Ser Lys Gln Met Glu Glu
    1               5                  10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 2
   <211> 39
   <212> PRT
   <213> Heloderma suspectum
<220>
   <221> AMIDATION
   <222> (39)...(39)
<223> amidated Ser (Serinamide)
<400> 2
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 3
   <211> 30
   <212> PRT
   <213> Homo Sapien
<220>
   <221> AMIDATION
   <222> (30)...(30)
   <223> amidated Arg (Argininamide)
<400> 3
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg
   20 25 30
<210> 4
   <211> 29
   <212> PRT
   <213> Artificial Sequence
<220>
   <223> artificially synthesized sequence of novel exendin agonist compound
<220>
   <221> VARIANT
   <222> (1)...(7)
   <223> Xaa in position 1 is His, Arg or Tyr; Xaa in position 2 is Ser, Gly, Ala or Thr; Xaa in position 3 is Asp or Glu; Xaa in position 5 is Ala or Thr; Xaa in position 6 is Ala, Phe, Tyr or naphthylalanine; Xaa in position 7 is Thr or Ser;
<220>
   <221> VARIANT
   <222> (8)...(13)
   <223> Xaa in position 8 is Ala, Ser or Thr; Xaa in position 9 is Asp or Glu; Xaa in position 10 is Ala, Leu, Ile, Val, pentylglycine or Met; Xaa in position 11 is Ala or Ser; Xaa in position 12 is Ala or Lys; Xaa in position 13 is Ala or Gln;
<220>
   <221> VARIANT
   <222> (14)...(20)
   <223> Xaa in position 14 is Ala, Leu, Ile, pentylglycine, Val or Met; Xaa in position 15 is Ala or Glu; Xaa in position 16 is Ala or Glu; Xaa in position 17 is Ala or Glu; Xaa in position 19 is Ala or Val; Xaa in position 20 is Ala or Arg;
<220>
   <221> VARIANT
   <222> (21)...(24)
   <223> Xaa in position 21 is Ala or Leu; Xaa in position 22 is Phe, Tyr or naphthylalanine; Xaa in position 23 is Ile, Val, Leu, pentylglycine, tert-butylglycine or Met; Xaa in position 24 is Ala, Glu or Asp;
<220>
   <221> VARIANT
   <222> (25) ... (28)
   <223> Xaa in position 25 is Ala, Trp, Phe, Tyr or naphthylalanine; Xaa in position 26 is Ala or Leu; Xaa in position 27 is Ala or Lys; Xaa in position 28 is Ala or Asn;
<220>
   <221> VARIANT
   <222> (29)...(29)
   <223> Xaa in position 29 is -OH; -NH₂; Gly-Z₂; Gly Gly-Z₂; Gly Gly Xaa₃₁-Z_{2;} Gly Gly Xaa₃₁ Ser-Z₂; Gly Gly Xaa₃₁ Ser Ser-Z₂; Gly Gly Xaa₃₁ Ser Ser Gly-Z₂; Gly Gly Xaa₃₁ Ser Ser Gly Ala-Z₂; Gly Gly Xaa₃₁ Ser Ser Gly Ala Xaa₃₆-Z₂;
<220>
   <221> VARIANT
   <222> (29)...(29)
   <223> Gly Gly Xaa₃₁ Ser Ser Gly Ala Xaa₃₆ Xaa₃₇-Z₂; or Gly Gly Xaa₃₁ Ser Ser Gly Ala Xaa₃₆ Xaa₃₇ Xaa₃₈-Z₂;
<220>
   <221> VARIANT
   <222> (29)...(29)
   <223> where Xaa₃₁, Xaa₃₆, Xaa₃₇ and Xaa₃₈ are independently Pro, homoproline, 3Hyp, 4Hyp, thioproline, N-alkylglycine, N-alkylpentylglycine or N-alkylalanine; and Z₂ is -OH or -NH₂;
<220>
   <221> VARIANT
   <222> (3)...(28)
   <223> provided that no more than three of Xaa in positions 3, 5, 6, 8, 10, 11, 12, 13, 14, 15, 16, 17, 19, 20, 21, 24, 25, 26, 27 and 28 are Ala.
<400> 4
   Xaa Xaa Xaa Gly Xaa Xaa Xaa Xaa Xaa Xaa Xaa Xaa Xaa Xaa Xaa Xaa
   1               5                   10                  15
   Xaa Ala Xaa Xaa Xaa Xaa Xaa Xaa Xaa Xaa Xaa Xaa Xaa
               20                  25
<210> 5
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 5
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 6
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 6
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 7
   <211> 28
   <212> PRT
   <213> Artificial Sequence
<220>
<223> artificially synthesized sequence of novel exendin agonist compound
<220>
   <221> AMIDATION
   <222> (28)...(28)
   <223> amidated Asn (Asparaginamide)

<400> 7
   His Gly Glu Ala Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 8
   <211> 28
   <212> PRT
   <213> Artificial Sequence
<220>
   <223> artificially synthesized sequence of novel exendin agonist
compound
<220>
   <221> AMIDATION
   <222> (28)...(28)
   <223> amidated Asn (Asparaginamide)
<400> 8
   His Gly Glu Gly Thr Phe Thr Ser Ala Leu Ser Lys Gln Leu Glu Glu
    1               5                  10                  15
   Glu Ala Val Arg Leu Phe Ile Glu Phe Leu Lys Asn
               20                  25
<210> 9
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 9
   Ala Gly Glu Gly Thr Phe Thr Ser Asp Leu Ser Lys Gln Met Glu Glu
    1               5                  10                  15
   Glu Ala Val Arg Leu Phe Ile Glu Trp Leu Lys Asn
               20                  25
<210> 10
   <211> 28
   <212> PRT
   <213> Artificial Sequence
   <220>
   <223> artificially synthesized sequence of novel exendin agonist compound
<220>
   <221> AMIDATION
   <222> (28)...(28)
   <223> amidated Asn (Asparaginamide)
<400> 10
   His Gly Ala Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 11
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 11
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 12
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 12
   His Gly Glu Gly Thr Phe Thr Ser Ala Leu Ser Lys Gln Met Glu Glu
   1 5 10 15
   Glu Ala Val Arg Leu Phe Ile Glu Trp Leu Lys Asn
   20 25
<210> 13
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 13
   His Gly Glu Gly Thr Phe Thr Ser Asp Ala Ser Lys Gln Met Glu Glu
    1               5                  10                  15
   Glu Ala Val Arg Leu Phe Ile Glu Trp Leu Lys Asn
               20                  25
<210> 14
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 14
   Ala Ala Glu Gly Thr Phe Thr Ser Asp Leu Ser Lys Gln Met Glu Glu
    1               5                  10                  15
   Glu Ala Val Arg Leu Phe Ile Glu Trp Leu Lys Asn
               20                  25
<210> 15
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 15
   Ala Ala Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 16
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 16
   Ala Gly Asp Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 17
<211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 17
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 18
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 18
   Ala Gly Asp Gly Ala Phe Thr Ser Asp Leu Ser Lys Gln Met Glu Glu
    1               5                   10                  15
   Glu Ala Val Arg Leu Phe Ile Glu Trp Leu Lys Asn
               20                  25
<210> 19
   <211> 28
   <212> PRT
   <213> Artificial Sequence
<220>
   <223> artificially synthesized sequence of novel exendin agonist compound
<220>
   <221> AMIDATION
<222> (28)...(28)
   <223> amidated Asn (Asparaginamide)
<400> 19
   Ala Gly Asp Gly Ala Phe Thr Ser Asp Leu Ser Lys Gln Leu Glu Glu
    1               5                   10                  15
   Glu Ala Val Arg Leu Phe Ile Glu Phe Leu Lys Asn
               20                  25
<210> 20
   <211> 28
   <212> PRT
   <213> Artificial Sequence
<220>
   <223> artificially synthesized sequence of novel exendin agonist compound
<220>
   <223> Xaa in position 6 stands for naphthylalanine.
<220>
   <221> AMIDATION
   <222> (28)...(28)
   <223> amidated Asn (Asparaginamide)
<400> 20
   Ala Gly Asp Gly Thr Xaa Thr Ser Asp Leu Ser Lys Gln Met Glu Glu
   1               5                   10                  15
   Glu Ala Val Arg Leu Phe Ile Glu Trp Leu Lys Asn
               20                  25
<210> 21
   <211> 28
   <212> PRT
   <213> Artificial Sequence
<220>
   <223> artificially synthesized sequence of novel exendin agonist compound
<220>
   <223> Xaa in position 6 stands for naphthylalanine.
<220>
   <221> AMIDATION
   <222> (28)...(28)
   <223> amidated Asn (Asparaginamide)
<400> 21
   Ala Gly Asp Gly Thr Xaa Thr Ser Asp Leu Ser Lys Gln Leu Glu Glu
   1               5                   10                  15
   Glu Ala Val Arg Leu Phe Ile Glu Phe Leu Lys Asn
               20                  25
<210> 22
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 22
   Ala Gly Asp Gly Thr Phe Ser Ser Asp Leu Ser Lys Gln Met Glu Glu
   1               5                   10                  15
   Glu Ala Val Arg Leu Phe Ile Glu Trp Leu Lys Asn
               20                  25
<210> 23
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 23
   Ala Gly Asp Gly Thr Phe Ser Ser Asp Leu Ser Lys Gln Leu Glu Glu
    1               5                   10                  15
   Glu Ala Val Arg Leu Phe Ile Glu Phe Leu Lys Asn
               20                  25
<210> 24
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 24
   Ala Gly Asp Gly Thr Phe Thr Ala Asp Leu Ser Lys Gln Met Glu Glu
    1               5                   10                  15
   Glu Ala Val Arg Leu Phe Ile Glu Trp Leu Lys Asn
               20                  25
<210> 25
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 25
   Ala Gly Asp Gly Thr Phe Thr Ala Asp Leu Ser Lys Gln Leu Glu Glu
   1 5 10 15
   Glu Ala Val Arg Leu Phe Ile Glu Phe Leu Lys Asn
   20 25
<210> 26
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 26
   Ala Gly Asp Gly Thr Phe Thr Ser Ala Leu Ser Lys Gln Met Glu Glu
   1 5 10 15
   Glu Ala Val Arg Leu Phe Ile Glu Trp Leu Lys Asn
   20 25
<210> 27
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 27
   Ala Gly Asp Gly Thr Phe Thr Ser Ala Leu Ser Lys Gln Leu Glu Glu
   1 5 10 15
   Glu Ala Val Arg Leu Phe Ile Glu Phe Leu Lys Asn
   20 25
<210> 28
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 28
   Ala Gly Asp Gly Thr Phe Thr Ser Glu Leu Ser Lys Gln Met Glu Glu
    1               5                  10                  15
   Glu Ala Val Arg Leu Phe Ile Glu Trp Leu Lys Asn
               20                  25
<210> 29
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 29
   Ala Gly Asp Gly Thr Phe Thr Ser Glu Leu Ser Lys Gln Leu Glu Glu
    1               5                  10                  15
   Glu Ala Val Arg Leu Phe Ile Glu Phe Leu Lys Asn
               20                  25
<210> 30
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 30
   Ala Gly Asp Gly Thr Phe Thr Ser Asp Ala Ser Lys Gln Met Glu Glu
   1 5 10 15
   Glu Ala Val Arg Leu Phe Ile Glu Trp Leu Lys Asn
   20 25
<210> 31
   <211> 28
   <212> PRT
   <213> Artificial Sequence
<220>
<223> artificially synthesized sequence of novel exendin agonist compound
<220>
   <221> AMIDATION
   <222> (28)...(28)
   <223> amidated Asn (Asparaginamide)

<400> 31
   Ala Gly Asp Gly Thr Phe Thr Ser Asp Ala Ser Lys Gln Leu Glu Glu
   1 5 10 15
   Glu Ala Val Arg Leu Phe Ile Glu Phe Leu Lys Asn
   20 25
<210> 32
   <211> 28
   <212> PRT
   <213> Artificial Sequence
<220>
   <223> artificially synthesized sequence of novel exendin agonist compound
<220>
   <223> Xaa in position 10 stands for pentylglycine.
<220>
   <221> AMIDATION
   <222> (28)...(28)
   <223> amidated Asn (Asparaginamide)
<400> 32
   Ala Gly Asp Gly Thr Phe Thr Ser Asp Xaa Ser Lys Gln Met Glu Glu
   1 5 10 15
   Glu Ala Val Arg Leu Phe Ile Glu Trp Leu Lys Asn
   20 25
<210> 33
   <211> 28
   <212> PRT
   <213> Artificial Sequence
<220>
   <223> artificially synthesized sequence of novel exendin agonist compound
<220>
   <223> Xaa in position 10 stands for pentylglycine.
<220>
   <221> AMIDATION
   <222> (28)...(28)
   <223> amidated Asn (Asparaginamide)
<400> 33
   Ala Gly Asp Gly Thr Phe Thr Ser Asp Xaa Ser Lys Gln Leu Glu Glu
    1              5                   10                  15
   Glu Ala Val Arg Leu Phe Ile Glu Phe Leu Lys Asn
               20                  25
<210> 34
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 34
   Ala Gly Asp Gly Thr Phe Thr Ser Asp Leu Ala Lys Gln Met Glu Glu
    1              5                   10                  15
   Glu Ala Val Arg Leu Phe Ile Glu Trp Leu Lys Asn
               20                  25
<210> 35
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 35
   Ala Gly Asp Gly Thr Phe Thr Ser Asp Leu Ala Lys Gln Leu Glu Glu
   1 5 10 15
   Glu Ala Val Arg Leu Phe Ile Glu Phe Leu Lys Asn
   20 25
<210> 36
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 36
   Ala Gly Asp Gly Thr Phe Thr Ser Asp Leu Ser Ala Gln Met Glu Glu
   1 5 10 15
   Glu Ala Val Arg Leu Phe Ile Glu Trp Leu Lys Asn
   20 25
<210> 37
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 37
   Ala Gly Asp Gly Thr Phe Thr Ser Asp Leu Ser Ala Gln Leu Glu Glu
   1 5 10 15
   Glu Ala Val Arg Leu Phe Ile Glu Phe Leu Lys Asn
   20 25
<210> 38
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 38
   Ala Gly Asp Gly Thr Phe Thr Ser Asp Leu Ser Lys Ala Met Glu Glu
   1 5 10 15
   Glu Ala Val Arg Leu Phe Ile Glu Trp Leu Lys Asn
   20 25
<210> 39 <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 39
   Ala Gly Asp Gly Thr Phe Thr Ser Asp Leu Ser Lys Ala Leu Glu Glu
   1 5 10 15
   Glu Ala Val Arg Leu Phe Ile Glu Phe Leu Lys Asn
   20 25
<210> 40 <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 40
   Ala Gly Asp Gly Thr Phe Thr Ser Asp Leu Ser Lys Gln Ala Glu Glu
   1               5                   10                  15
   Glu Ala Val Arg Leu Phe Ile Glu Trp Leu Lys Asn
               20                  25
<210> 41 <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 41
   Ala Gly Asp Gly Thr Phe Thr Ser Asp Leu Ser Lys Gln Ala Glu Glu
   1               5                   10                  15
   Glu Ala Val Arg Leu Phe Ile Glu Phe Leu Lys Asn
               20                  25
<210> 42 <211> 28
   <212> PRT
   <213> Artificial Sequence
<220>
   <223> artificially synthesized sequence of novel exendin agonist compound
<220>
   <223> Xaa in position 14 stands for pentylglycine.
<220>
   <221> AMIDATION
   <222> (28)...(28)
   <223> amidated Asn (Asparaginamide)
<400> 42
   Ala Gly Asp Gly Thr Phe Thr Ser Asp Leu Ser Lys Gln Xaa Glu Glu
   1 5 10 15
   Glu Ala Val Arg Leu Phe Ile Glu Trp Leu Lys Asn
   20 25
<210> 43 <211> 28
   <212> PRT
   <213> Artificial Sequence
<220>
   <223> artificially synthesized sequence of novel exendin agonist compound
<220>
   <223> Xaa in position 14 stands for pentylglycine.
<220>
   <221> AMIDATION
   <222> (28)...(28)
   <223> amidated Asn (Asparaginamide)
<400> 43
   Ala Gly Asp Gly Thr Phe Thr Ser Asp Leu Ser Lys Gln Xaa Glu Glu
   1 5 10 15
   Glu Ala Val Arg Leu Phe Ile Glu Phe Leu Lys Asn
   20 25
<210> 44 <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 44
   Ala Gly Asp Gly Thr Phe Thr Ser Asp Leu Ser Lys Gln Met Ala Glu
   1 5 10 15
   Glu Ala Val Arg Leu Phe Ile Glu Trp Leu Lys Asn
   20 25
<210> 45 <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 45
   Ala Gly Asp Gly Thr Phe Thr Ser Asp Leu Ser Lys Gln Leu Ala Glu
   1               5                   10                  15
   Glu Ala Val Arg Leu Phe Ile Glu Phe Leu Lys Asn
               20                  25
<210> 46
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 46
   Ala Gly Asp Gly Thr Phe Thr Ser Asp Leu Ser Lys Gln Met Glu Ala
   1               5                   10                  15
   Glu Ala Val Arg Leu Phe Ile Glu Trp Leu Lys Asn
               20                  25
<210> 47
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 47
   Ala Gly Asp Gly Thr Phe Thr Ser Asp Leu Ser Lys Gln Leu Glu Ala
   1 5 10 15
   Glu Ala Val Arg Leu Phe Ile Glu Phe Leu Lys Asn
   20 25
<210> 48
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 48
   Ala Gly Asp Gly Thr Phe Thr Ser Asp Leu Ser Lys Gln Met Glu Glu
   1 5 10 15
   Ala Ala Val Arg Leu Phe Ile Glu Trp Leu Lys Asn
   20 25
<210> 49
   <211> 28
   <212> PRT
   <213> Artificial Sequence
<220>
   <223> artificially synthesized sequence of novel exendin agonist compound
<220>
   <221> AMIDATION
   <222> (28)... (28)
   <223> amidated Asn (Asparaginamide)
<400> 49
   Ala Gly Asp Gly Thr Phe Thr Ser Asp Leu Ser Lys Gln Leu Glu Glu
   1 5 10 15
   Ala Ala Val Arg Leu Phe Ile Glu Phe Leu Lys Asn
   20 25
<210> 50
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 50
   Ala Gly Asp Gly Thr Phe Thr Ser Asp Leu Ser Lys Gln Met Glu Glu
   1               5                   10                  15
   Glu Ala Ala Arg Leu Phe Ile Glu Trp Leu Lys Asn
               20                  25
<210> 51
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 51
   Ala Gly Asp Gly Thr Phe Thr Ser Asp Leu Ser Lys Gln Leu Glu Glu
   1               5                   10                  15
   Glu Ala Ala Arg Leu Phe Ile Glu Phe Leu Lys Asn
               20                  25
<210> 52
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 52
   Ala Gly Asp Gly Thr Phe Thr Ser Asp Leu Ser Lys Gln Met Glu Glu
   1 5 10 15
   Glu Ala Val Ala Leu Phe Ile Glu Trp Leu Lys Asn
   20 25
<210> 53
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 53
   Ala Gly Asp Gly Thr Phe Thr Ser Asp Leu Ser Lys Gln Leu Glu Glu
   1 5 10 15
   Glu Ala Val Ala Leu Phe Ile Glu Phe Leu Lys Asn
   20 25
<210> 54
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)

<400> 54
   Ala Gly Asp Gly Thr Phe Thr Ser Asp Leu Ser Lys Gln Met Glu Glu
   1 5 10 15
   Glu Ala Val Arg Ala Phe Ile Glu Trp Leu Lys Asn
   20 25
<210> 55
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 55
   Ala Gly Asp Gly Thr Phe Thr Ser Asp Leu Ser Lys Gln Leu Glu Glu
   1               5                   10                  15
   Glu Ala Val Arg Ala Phe Ile Glu Phe Leu Lys Asn
               20                  25
<210> 56
   <211> 28
   <212> PRT
   <213> Artificial Sequence
<220>
   <223> artificially synthesized sequence of novel exendin agonist compound
<220>
   <223> Xaa in position 22 stands for naphthylalanine.
<220>
   <221> AMIDATION
   <222> (28)...(28)
   <223> amidated Asn (Asparaginamide)
<400> 56
   Ala Gly Asp Gly Thr Phe Thr Ser Asp Leu Ser Lys Gln Met Glu Glu
   1               5                   10                  15
   Glu Ala Val Arg Leu Xaa Ile Glu Trp Leu Lys Asn
               20                  25
<210> 57
   <211> 28
   <212> PRT
   <213> Artificial Sequence
<220>
   <223> artificially synthesized sequence of novel exendin agonist compound
<220>
   <223> Xaa in position 22 stands for naphthylalanine.
<220>
   <221> AMIDATION
   <222> (28)...(28)
   <223> amidated Asn (Asparaginamide)
<400> 57
   Ala Gly Asp Gly Thr Phe Thr Ser Asp Leu Ser Lys Gln Leu Glu Glu
   1 5 10 15
   Glu Ala Val Arg Leu Xaa Ile Glu Phe Leu Lys Asn
   20 25
<210> 58
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 58
   Ala Gly Asp Gly Thr Phe Thr Ser Asp Leu Ser Lys Gln Met Glu Glu
   1 5 10 15
   Glu Ala Val Arg Leu Phe Val Glu Trp Leu Lys Asn
   20 25
<210> 59
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 59
   Ala Gly Asp Gly Thr Phe Thr Ser Asp Leu Ser Lys Gln Leu Glu Glu
   1 5 10 15
   Glu Ala Val Arg Leu Phe Val Glu Phe Leu Lys Asn
   20 25
<210> 60
   <211> 28
   <212> PRT
   <213> Artificial Sequence
<220>
   <223> artificially synthesized sequence of novel exendin agonist compound
<220>
   <223> Xaa in position 23 stands for tert-butylglycine.
<220>
   <221> AMIDATION
   <222> (28)...(28)
   <223> amidated Asn (Asparaginamide)
<400> 60
   Ala Gly Asp Gly Thr Phe Thr Ser Asp Leu Ser Lys Gln Met Glu Glu
   1 5 10 15
   Glu Ala Val Arg Leu Phe Xaa Glu Trp Leu Lys Asn
   20 25
<210> 61
   <211> 28
   <212> PRT
   <213> Artificial Sequence
<220>
   <223> artificially synthesized sequence of novel exendin agonist compound
<220>
   <223> Xaa in position 23 stands for tert-butylglycine.
<220>
   <221> AMIDATION
   <222> (28) ... (28)
   <223> amidated Asn (Asparaginamide)
<400> 61
   Ala Gly Asp Gly Thr Phe Thr Ser Asp Leu Ser Lys Gln Leu Glu Glu
   1 5 10 15
   Glu Ala Val Arg Leu Phe Xaa Glu Phe Leu Lys Asn
   20 25
<210> 62
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 62
   Ala Gly Asp Gly Thr Phe Thr Ser Asp Leu Ser Lys Gln Met Glu Glu
   1               5                   10                  15
   Glu Ala Val Arg Leu Phe Ile Asp Trp Leu Lys Asn
               20                  25
<210> 63
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 63
   Ala Gly Asp Gly Thr Phe Thr Ser Asp Leu Ser Lys Gln Leu Glu Glu
   1               5                   10                  15
   Glu Ala Val Arg Leu Phe Ile Asp Phe Leu Lys Asn
               20                  25
<210> 64
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 64
   Ala Gly Asp Gly Thr Phe Thr Ser Asp Leu Ser Lys Gln Met Glu Glu
   1 5 10 15
   Glu Ala Val Arg Leu Phe Ile Glu Ala Leu Lys Asn
   20 25
<210> 65
   <211> 28
<212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 65
   Ala Gly Asp Gly Thr Phe Thr Ser Asp Leu Ser Lys Gln Leu Glu Glu
   1 5 10 15
   Glu Ala Val Arg Leu Phe Ile Glu Ala Leu Lys Asn
   20 25
<210> 66
<211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 66
   Ala Gly Asp Gly Thr Phe Thr Ser Asp Leu Ser Lys Gln Met Glu Glu
   1 5 10 15
   Glu Ala Val Arg Leu Phe Ile Glu Trp Ala Lys Asn
   20 25
<210> 67
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 67
   Ala Gly Asp Gly Thr Phe Thr Ser Asp Leu Ser Lys Gln Leu Glu Glu
    1               5                   10                  15
   Glu Ala Val Arg Leu Phe Ile Glu Phe Ala Lys Asn
               20                  25
<210> 68
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 68
   Ala Gly Asp Gly Thr Phe Thr Ser Asp Leu Ser Lys Gln Met Glu Glu
    1               5                   10                  15
   Glu Ala Val Arg Leu Phe Ile Glu Trp Leu Ala Asn
               20                  25
<210> 69
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Asn (Asparaginamide)
<400> 69
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Ala Asn
   20 25
<210> 70
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Ala (Alaninamide)
<400> 70
   Ala Gly Asp Gly Thr Phe Thr Ser Asp Leu Ser Lys Gln Met Glu Glu
   1 5 10 15
   Glu Ala Val Arg Leu Phe Ile Glu Trp Leu Lys Ala
   20 25
<210> 71
   <211> 28
   <212> PRT
   <213> Artificial Sequence
<220>
   <223> artificially synthesized sequence of novel exendin agonist compound
<220>
   <221> AMIDATION
   <222> (28)...(28)
   <223> amidated Ala (Alaninamide)
<400> 71
   Ala Gly Asp Gly Thr Phe Thr Ser Asp Leu Ser Lys Gln Leu Glu Glu
   1 5 10 15
   Glu Ala Val Arg Leu Phe Ile Glu Phe Leu Lys Ala
   20 25
<210> 72
   <211> 38
   <212> PRT
   <213> Artificial Sequence
<220>
   <223> artificially synthesized sequence of novel exendin agonist compound
<220>
   <221> AMIDATION
   <222> (38)...(38)
   <223> amidated Pro (Prolinamide)
<400> 72
   Ala Gly Glu Gly Thr Phe Thr Ser Asp Leu Ser Lys Gln Met Glu Glu
    1               5                  10                  15
   Glu Ala Val Arg Leu Phe Ile Glu Trp Leu Lys Asn Gly Gly Pro Ser
               20                  25                  30
   Ser Gly Ala Pro Pro Pro
           35
<210> 73
   <211> 38
   <212> PRT
   <213> Artificial Sequence
<220>
   <223> artificially synthesized sequence of novel exendin agonist compound
<220>
   <221> AMIDATION
   <222> (38)...(38)
   <223> amidated amidated Pro (Prolinamide)
<400> 73
   His Gly Ala Gly Thr Phe Thr Ser Asp Leu Ser Lys Gln Leu Glu Glu
    1               5                  10                  15
   Glu Ala Val Arg Leu Phe Ile Glu Phe Leu Lys Asn Gly Gly Pro Ser
               20                  25                  30
   Ser Gly Ala Pro Pro Pro
           35
<210> 74
   <211> 37
   <212> PRT
   <213> Artificial Sequence
<220>
   <223> artificially synthesized sequence of novel exendin agonist compound
<220>
   <221> AMIDATION
   <222> (37)...(37)
   <223> amidated amidated Pro (Prolinamide)
<400> 74
   His Gly Glu Ala Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro
           35
<210> 75
   <211> 36
   <212> PRT
   <213> Artificial Sequence
<220>
   <223> artificially synthesized sequence of novel exendin agonist compound
<220>
   <221> AMIDATION
   <222> (36)...(36)
   <223> amidated amidated Pro (Prolinamide)
<400> 75
   His Gly Glu Gly Thr Phe Thr Ser Ala Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro
           35
<210> 76
   <211> 36
   <212> PRT
   <213> Artificial Sequence
<220>
   <223> artificially synthesized sequence of novel exendin agonist compound
<220>
   <221> AMIDATION
   <222> (36)...(36)
   <223> amidated Pro (Prolinamide)
<400> 76
   Ala Gly Glu Gly Thr Phe Thr Ser Asp Ala Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro
   35
<210> 77
   <211> 35
   <212> PRT
   <213> Artificial Sequence
<220>
   <223> artificially synthesized sequence of novel exendin agonist compound
<220>
   <221> AMIDATION
   <222> (35)...(35)
   <223> amidated Ala (Alaninamide)

<400> 77
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala
   35
<210> 78
   <211> 35
   <212> PRT
   <213> Artificial Sequence
<220>
   <223> artificially synthesized sequence of novel exendin agonist compound
<220>
   <221> AMIDATION
   <222> (35)...(35)
   <223> amidated Ala (Alaninamide)
<400> 78
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala
   35
<210> 79
   <211> 34
   <212> PRT
   <213> Artificial Sequence
<220>
   <223> artificially synthesized sequence of novel exendin agonist compound
<220>
   <221> AMIDATION
   <222> (34)...(34)
   <223> amidated Gly (Glycinamide)
<400> 79
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly
<210> 80
   <211> 33
   <212> PRT
   <213> Artificial Sequence
<220>
   <223> artificially synthesized sequence of novel exendin agonist compound
<220>
   <221> AMIDATION
   <222> (33)...(33)
   <223> amidated Ser (Serinamide)
<400> 80
   His Gly Glu Gly Thr Phe Thr Ser Ala Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser
<210> 81
   <211> 32
   <212> PRT
   <213> Artificial Sequence
<220>
   <223> artificially synthesized sequence of novel exendin agonist compound
<220>
   <221> AMIDATION
   <222> (32)...(32)
   <223> amidated Ser (Serinamide)
<400> 81
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
<210> 82
   <211> 32
   <212> PRT
   <213> Artificial Sequence
<220>
   <223> artificially synthesized sequence of novel exendin agonist compound
<220>
   <221> AMIDATION
   <222> (32)...(32)
   <223> amidated Ser (Serinamide)
<400> 82
   His Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
<210> 83
   <211> 31
   <212> PRT
   <213> Artificial Sequence
<220>
   <223> artificially synthesized sequence of novel exendin agonist compound
<220>
   <221> AMIDATION
   <222> (31)...(31)
   <223> amidated Pro (Prolinamide)
<400> 83
   His Gly Glu Ala Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro
   20 25 30
<210> 84
   <211> 30
   <212> PRT
   <213> Artificial Sequence
<220>
   <223> artificially synthesized sequence of novel exendin agonist compound
<220>
   <221> AMIDATION
   <222> (30)...(30)
   <223> amidated Gly (Glycinamide)
<400> 84
   His Gly Glu Gly Thr Phe Thr Ser Ala Leu Ser Lys Gln Leu Glu Glu
   1 5 10 15
   Glu Ala Val Arg Leu Phe Ile Glu Phe Leu Lys Asn Gly Gly
   20 25 30
<210> 85
   <211> 29
   <212> PRT
   <213> Artificial Sequence
<220>
   <223> artificially synthesized sequence of novel exendin agonist compound
<220>
   <221> AMIDATION
   <222> (29)...(29)
   <223> amidated Gly (Glycinamide)
<400> 85
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly
   20 25
<210> 86
   <211> 38
   <212> PRT
   <213> Artificial Sequence
<220>
   <223> artificially synthesized sequence of novel exendin agonist compound
<220>
   <223> Xaa in positions 31, 36, 37 and 38 stands for thioproline.
<220>
   <221> AMIDATION
   <222> (38)...(38)
   <223> amidated tPro (Thioprolinamide)
<400> 86
   His Gly Ala Gly Thr Phe Thr Ser Asp Leu Ser Lys Gln Met Glu Glu
    1               5                  10                  15
   Glu Ala Val Arg Leu Phe Ile Glu Trp Leu Lys Asn Gly Gly Xaa Ser
               20                  25                  30
   Ser Gly Ala Xaa Xaa Xaa
           35
<210> 87
   <211> 38
   <212> PRT
   <213> Artificial Sequence
<220>
   <223> artificially synthesized sequence of novel exendin agonist compound
<220>
   <223> Xaa in positions 36, 37 and 38 stands for thioproline.
<220>
   <221> AMIDATION
   <222> (38)...(38)
   <223> amidated tPro (Thioprolinamide)
<400> 87
   His Gly Glu Ala Thr Phe Thr Ser Asp Leu Ser Lys Gln Met Glu Glu
    1               5                  10                  15
   Glu Ala Val Arg Leu Phe Ile Glu Trp Leu Lys Asn Gly Gly Pro Ser
               20                  25                  30
   Ser Gly Ala Xaa Xaa Xaa
           35
<210> 88
   <211> 37
   <212> PRT
   <213> Artificial Sequence
<220>
   <223> artificially synthesized sequence of novel exendin agonist compound
<220>
   <223> Xaa in positions 31, 36 and 37 stands for N-methyl ala.
<220>
   <221> AMIDATION
   <222> (37)...(37)
   <223> amidated N-methyl ala (N-methyl alaninamide)
<400> 88
   His Gly Glu Gly Thr Phe Thr Ser Ala Leu Ser Lys Gln Met Glu Glu
    1               5                  10                  15
   Glu Ala Val Arg Leu Phe Ile Glu Trp Leu Lys Asn Gly Gly Xaa Ser
               20                  25                  30
   Ser Gly Ala Xaa Xaa
           35
<210> 89
   <211> 36
   <212> PRT
   <213> Artificial Sequence
<220>
   <223> artificially synthesized sequence of novel exendin agonist compound
<220>
   <223> Xaa in positions 31 and 36 stands for homoproline.
<220>
   <221> AMIDATION
   <222> (36)...(36)
   <223> amidated hPro (Homoprolinamide)
<400> 89
   Ala Gly Glu Gly Thr Phe Thr Ser Asp Leu Ser Lys Gln Met Glu Glu
    1               5                  10                  15
   Glu Ala Val Arg Leu Phe Ile Glu Trp Leu Lys Asn Gly Gly Xaa Ser
               20                  25                  30
   Ser Gly Ala Xaa
           35
<210> 90
   <211> 35
   <212> PRT
   <213> Artificial Sequence
<220>
   <223> artificially synthesized sequence of novel exendin agonist compound
<220>
   <221> AMIDATION
   <222> (35)...(35)
   <223> amidated Ala (Alaninamide)
<400> 90
   His Gly Ala Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala
           35
<210> 91
   <211> 30
   <212> PRT
   <213> Artificial Sequence
<220>
   <223> artificially synthesized sequence of novel exendin agonist compound
<220>
   <221> AMIDATION
   <222> (30)...(30)
   <223> amidated Gly (Glycinamide)
<400> 91
   His Gly Asp Ala Thr Phe Thr Ser Asp Leu Ser Lys Gln Met Glu Glu
   1               5                   10                  15
   Glu Ala Val Arg Leu Phe Ile Glu Trp Leu Lys Asn Gly Gly
               20                  25                  30
<210> 92
   <211> 39
   <212> PRT
   <213> Artificial Sequence
<220>
   <223> artificially synthesized sequence of novel exendin agonist compound
<220>
   <221> AMIDATION
   <222> (39)...(39)
   <223> amidated Ser (Serinamide)
<400> 92
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 93
   <211> 39
   <212> PRT
   <213> Artificial Sequence
<220>
   <223> artificially synthesized sequence of novel exendin agonist compound
<220>
   <221> AMIDATION
   <222> (39)...(39)
   <223> amidated Ser (Serinamide)
<400> 93
   Ala Gly Ala Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 94
   <211> 28
   <212> PRT
   <213> Artificial Sequence
<220>
   <223> artificially synthesized sequence of novel exendin agonist compound
<220>
   <221> VARIANT
   <222> (1) ... (4)
   <223> Xaa in position 1 is His, Arg, Tyr, Ala, Norval, Val, Norleu or 4-imidazopropionyl; Xaa in position 2 is Ser, Gly, Ala or Thr; Xaa in position 3 is Ala, Asp or Glu; Xaa in position 4 is Ala, Norval, Val, Norleu or Gly;
<220>
   <221> VARIANT
   <222> (5)...(9)
   <223> Xaa in position 5 is Ala or Thr; Xaa in position 6 is Phe, Tyr or naphthylalanine;Xaa in position 7 is Thr or Ser; Xaa in position 8 is Ala, Ser or Thr; Xaa in position 9 is Ala, Norval, Val, Norleu, Asp or Glu;
<220>
   <221> VARIANT
   <222> (10)...(14)
   <223> Xaa in position 10 is Ala, Leu, Ile, Val, pentylglycine or Met; Xaa in position 11 is Ala or Ser; Xaa in position 12 is Ala or Lys; Xaa in position 13 is Ala or Gln; Xaa in position 14 is Ala, Leu, Ile, pentylglycine, Val or Met;
<220>
   <221> VARIANT
   <222> (15)...(19)
   <223> Xaa in position 15 is Ala or Glu; Xaa in position 16 is Ala or Glu; Xaa in position 17 is Ala or Glu; Xaa in position 19 is Ala or Val;
<220>
   <221> VARIANT
   <222> (20)...(22)
   <223> Xaa in position 20 is Ala or Arg; Xaa in position 21 is Ala, Leu or Lys-NH^{ε}-R where R is Lys, Arg, C-C₁₀ straight chain or branched alkanoyl or cycloalkyl-alkanoyl; Xaa in position 22 is Phe, Tyr or naphthylalanine;
<220>
   <221> VARIANT
   <222> (23)...(26)
   <223> Xaa in position 23 is Ile, Val, Leu, pentylglycine, tert-butylglycine or Met; Xaa in position 24 is Ala, Glu or Asp; Xaa in position 25 is Ala, Trp, Phe, Tyr or naphthylalanine; Xaa in position 26 is Ala or Leu;
<220>
   <221> VARIANT
   <222> (27...(27)
   <223> Xaa in position 27 is Lys Asn, Asn Lys, Lys-NH^{ε}-R Asn, Asn Lys-NH^{ε}-R, Lys-NH^{ε}-R Ala, Ala Lys-NH^{ε}-R where R is Lys, Arg, C₁-C₁₀ straight chain or branched alkanoyl or cycloalkyl-alkanoyl;
<220>
   <221> VARIANT
   <222> (28) ... (28)
   <223> Xaa in position 28 is -OH; -NH₂; Gly-Z₂; Gly Gly-Z₂; Gly Gly Xaa₃₁-Z₂; Gly Gly Xaa₃₁ Ser-Z₂; Gly Gly Xaa₃₁ Ser Ser-Z₂; Gly Gly Xaa₃₁ Ser Ser Gly-Z₂; Gly Gly Xaa₃₁ Ser Ser Gly Ala-Z₂; Gly Gly Xaa₃₁ Ser Ser Gly Ala Xaa₃₆-Z₂;
<220>
   <221> VARIANT
   <222> (28)...(28)
   <223> Xaa in position 28 is Gly Gly Xaa₃₁ Ser Ser Gly Ala Xaa₃₆ Xaa₃₇-Z₂; Gly Gly Xaa₃₁ Ser Ser Gly Ala Xaa₃₆ Xaa₃₇ Xaa₃₈-Z₂; or
<220>
   <221> VARIANT
   <222> (28)...(28)
   <223> Xaa in position 28 is Gly Gly Xaa₃₁ Ser Ser Gly Ala Xaa₃₆ Xaa₃₇ Xaa₃₈ Xaa₃₉-Z₂;
<220>
   <221> VARIANT
   <222> (28)...(28)
   <223> wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; and Z₂ is -OH or -NH₂;
<220>
   <221> VARIANT
   <222> (3)...(26)
   <223> provided that no more than three of Xaa in positions 3, 4, 5, 6, 8, 9, 10, 11, 12, 13, 14, 15, 16, 17, 19, 20, 21, 24, 25 and 26 are Ala
<220>
   <221> VARIANT
   <222> (3)...(26)
   <223> and provided also that, if Xaa₁ is His, Arg, Tyr, or 4-imidazopropionyl then at least one of Xaa₃, Xaa₄ and Xaa₉ is Ala.
<400> 94
   Xaa Xaa Xaa Xaa Xaa Xaa Xaa Xaa Xaa Xaa Xaa Xaa Xaa Xaa Xaa Xaa
    1               5                  10                  15
   Xaa Ala Xaa Xaa Xaa Xaa Xaa Xaa Xaa Xaa Xaa Xaa
               20                  25
<210> 95
   <211> 27
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 26 is Lys-NH^{ε}octanoyl.
<220>
   <221> AMIDATION
   <222> (27)...(27)
   <223> amidated Asn (Asparaginamide)

<400> 95
   Xaa Glu Gly Thr Phe Thr Ser Ala Leu Ser Lys Gln Met Glu Glu Glu
    1               5                  10                  15
   Ala Val Arg Leu Phe Ile Glu Trp Leu Xaa Asn
               20                  25
<210> 96
   <211> 27
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 26 is Lys-NH^{ε}octanoyl.
<220>
   <221> AMIDATION
   <222> (27)...(27)
   <223> amidated Asn (Asparaginamide)
<400> 96
   Xaa Glu Gly Thr Phe Thr Ser Ala Leu Ser Lys Gln Leu Glu Glu Glu
   1 5 10 15
   Ala Val Arg Leu Phe Ile Glu Phe Leu Xaa Asn
   20 25
<210> 97
   <211> 29
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 26 is Lys-NH^{ε}octanoyl.
<220>
<221> AMIDATION
   <222> (29)...(29)
   <223> amidated Gly (Glycinamide)
<400> 97
   Xaa Glu Gly Thr Phe Thr Ser Ala Leu Ser Lys Gln Met Glu Glu Glu
   1 5 10 15
   Ala Val Arg Leu Phe Ile Glu Trp Leu Xaa Asn Gly Gly
   20 25
<210> 98
   <211> 29
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 26 is Lys-NH^{ε}octanoyl.
<220>
   <221> AMIDATION
   <222> (29)...(29)
   <223> amidated Gly (Glycinamide)
<400> 98
   Xaa Glu Gly Thr Phe Thr Ser Ala Leu Ser Lys Gln Leu Glu Glu Glu
   1 5 10 15
   Ala Val Arg Leu Phe Ile Glu Phe Leu Xaa Asn Gly Gly
   20 25
<210> 99
   <211> 27
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 27 is Lys-NH^{ε}octanoyl.
<220>
   <221> AMIDATION
   <222> (27)...(27)
   <223> amidated NH^{ε}octanoyl (NH^{ε}octanoylamide)
<400> 99
   Xaa Glu Gly Thr Phe Thr Ser Ala Leu Ser Lys Gln Met Glu Glu Glu
   1 5 10 15
   Ala Val Arg Leu Phe Ile Glu Trp Leu Asn Xaa
   20 25
<210> 100
   <211> 27
   <212> PRT
   <213> Artificial Sequence
<220>
   <223> artificially synthesized sequence of novel exendin agonist compound
<220> <223> Xaa in position 1 is 4-imidazolylpropionyl-Gly. Xaa in position 27 is Lys-NH^{ε}octanoyl.
<220>
   <221> AMIDATION
   <222> (27)...(27)
   <223> amidated NH^{ε}octanoyl (NH^{ε}octanoylamide)
<400> 100
   Xaa Glu Gly Thr Phe Thr Ser Ala Leu Ser Lys Gln Leu Glu Glu Glu
   1 5 10 15
   Ala Val Arg Leu Phe Ile Glu Phe Leu Asn Xaa
   20 25
<210> 101
   <211> 29
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 27 is Lys-NH^{ε}octanoyl.
<220>
   <221> AMIDATION
   <222> (29)...(29)
   <223> amidated Gly (Glycinamide)
<400> 101
   Xaa Glu Gly Thr Phe Thr Ser Ala Leu Ser Lys Gln Met Glu Glu Glu
   1 5 10 15
   Ala Val Arg Leu Phe Ile Glu Trp Leu Asn Xaa Gly Gly
   20 25
<210> 102
   <211> 29
   <212> PRT
   <213> Artificial Sequence
<220>
   <223> artificially synthesized sequence of novel exendin agonist compound
<220>
   <223> Xaa in position 1 is 4-imidazolylpropionyl-Gly. Xaa in position 27 is Lys-NH^{ε}octanoyl.
<220>
   <221> AMIDATION
   <222> (29)...(29)
   <223> amidated Gly (Glycinamide)
<400> 102
   Xaa Glu Gly Thr Phe Thr Ser Ala Leu Ser Lys Gln Leu Glu Glu Glu
   1 5 10 15
   Ala Val Arg Leu Phe Ile Glu Phe Leu Asn Xaa Gly Gly
   20 25
<210> 103
   <211> 28
   <212> PRT
   <213> Artificial Sequence
<220>
   <223> artificially synthesized sequence of novel exendin agonist compound
<220>
   <223> Xaa in position 27 is Lys-NH^{ε}octanoyl.
<220>
   <221> AMIDATION
   <222> (28)...(28)
   <223> amidated Asn (Asparaginamide)
<400> 103
   Ala Gly Glu Gly Thr Phe Thr Ser Asp Leu Ser Lys Gln Met Glu Glu
   1 5 10 15
   Glu Ala Val Arg Leu Phe Ile Glu Trp Leu Xaa Asn
   20 25
<210> 104
   <211> 28
   <212> PRT
   <213> Artificial Sequence
<220>
   <223> artificially synthesized sequence of novel exendin agonist compound
<220>
   <223> Xaa in position 27 is Lys-NH^{ε}octanoyl.
<220>
   <221> AMIDATION
   <222> (28)...(28)
   <223> amidated Asn (Asparaginamide)
<400> 104
   Ala Gly Glu Gly Thr Phe Thr Ser Asp Leu Ser Lys Gln Leu Glu Glu
   1 5 10 15
   Glu Ala Val Arg Leu Phe Ile Glu Phe Leu Xaa Asn
   20 25
<210> 105
   <211> 30
   <212> PRT
   <213> Artificial Sequence
<220>
   <223> artificially synthesized sequence of novel exendin agonist compound
<220>
   <223> Xaa in position 27 is Lys-NH^{ε}octanoyl.
<220>
   <221> AMIDATION
   <222> (30)...(30)
   <223> amidated Gly (Glycinamide)
<400> 105
   Ala Gly Glu Gly Thr Phe Thr Ser Asp Leu Ser Lys Gln Met Glu Glu
    1               5                  10                  15
   Glu Ala Val Arg Leu Phe Ile Glu Trp Leu Xaa Asn Gly Gly
               20                  25                  30
<210> 106
   <211> 30
   <212> PRT
   <213> Artificial Sequence
<220>
   <223> artificially synthesized sequence of novel exendin agonist compound
<220>
   <223> Xaa in position 27 is Lys-NH^{ε}octanoyl.
<220>
   <221> AMIDATION
   <222> (30)...(30)
   <223> amidated Gly (Glycinamide)
<400> 106
   Ala Gly Glu Gly Thr Phe Thr Ser Asp Leu Ser Lys Gln Leu Glu Glu
    1               5                  10                  15
   Glu Ala Val Arg Leu Phe Ile Glu Phe Leu Xaa Asn Gly Gly
               20                  25                  30
<210> 107
   <211> 28
   <212> PRT
   <213> Artificial Sequence
<220>
   <223> artificially synthesized sequence of novel exendin agonist compound
<220>
   <223> Xaa in position 28 is Lys-NH^{ε}octanoyl.
<220>
   <221> AMIDATION
   <222> (28)...(28)
   <223> amidated NH^{ε}octanoyl (NH^{ε}octanoylamide)
<400> 107
   Ala Gly Glu Gly Thr Phe Thr Ser Asp Leu Ser Lys Gln Met Glu Glu
    1               5                  10                  15
   Glu Ala Val Arg Leu Phe Ile Glu Trp Leu Asn Xaa
               20                  25
<210> 108
   <211> 28
   <212> PRT
   <213> Artificial Sequence
<220>
   <223> artificially synthesized sequence of novel exendin agonist compound
<220>
   <223> Xaa in position 28 is Lys-NH^{ε}octanoyl.
<220>
   <221> AMIDATION
<222> (28)...(28)
   <223> amidated NH^{ε}octanoyl (NH^{ε}octanoylamide)
<400> 108
   Ala Gly Glu Gly Thr Phe Thr Ser Asp Leu Ser Lys Gln Leu Glu Glu
    1               5                  10                  15
   Glu Ala Val Arg Leu Phe Ile Glu Phe Leu Asn Xaa
               20                  25
<210> 109
   <211> 30
   <212> PRT
   <213> Artificial Sequence
<220>
   <223> artificially synthesized sequence of novel exendin agonist compound
<220>
   <223> Xaa in position 28 is Lys-NH^{ε}octanoyl.
<220>
   <221> AMIDATION
   <222> (30)...(30)
   <223> amidated Gly (Glycinamide)
<400> 109
   Ala Gly Glu Gly Thr Phe Thr Ser Asp Leu Ser Lys Gln Met Glu Glu
    1              5                   10                  15
   Glu Ala Val Arg Leu Phe Ile Glu Trp Leu Asn Xaa Gly Gly
               20                  25                  30
<210> 110
   <211> 30
   <212> PRT
   <213> Artificial Sequence
<220>
   <223> artificially synthesized sequence of novel exendin agonist compound
<220>
   <223> Xaa in position 28 is Lys-NH^{ε} octanoyl.
<220>
   <221> AMIDATION
   <222> (30)...(30)
   <223> amidated Gly (Glycinamide)
   <400> 110
   Ala Gly Glu Gly Thr Phe Thr Ser Asp Leu Ser Lys Gln Leu Glu Glu
    1              5                   10                  15
   Glu Ala Val Arg Leu Phe Ile Glu Phe Leu Asn Xaa Gly Gly
               20                  25                  30

## Description

### Field of the Invention

The present invention relates to novel compounds which have activity as exendin agonists.

### BACKGROUND

The following description includes information that may be useful in understanding the present invention.

### Exendin

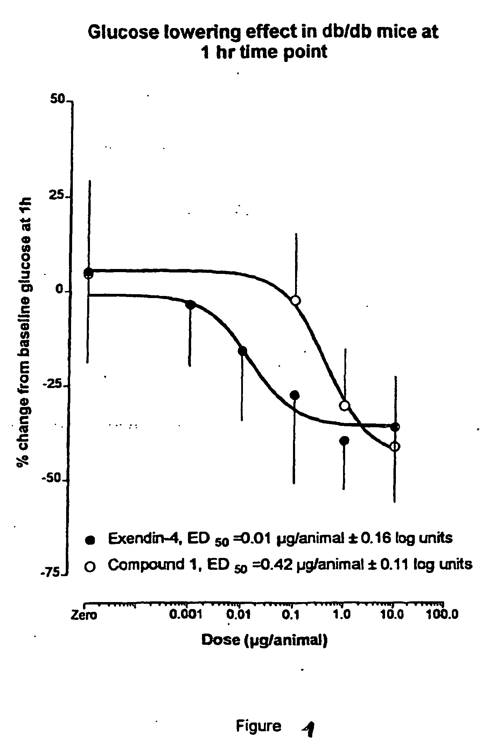

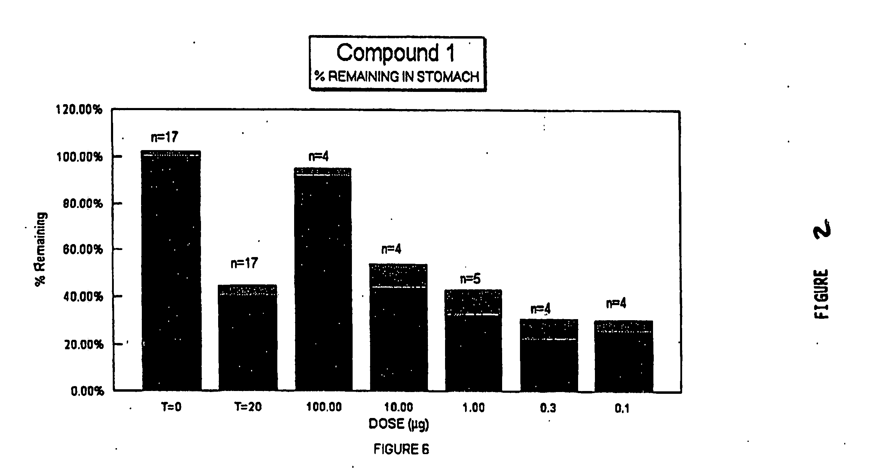

The exendins are peptides that are found in the venom of the Gila-monster, a lizard endogenous to Arizona and Northern-Mexico. Exendin-3 [SEQ. ID. NO. 1] is present in the venom of *Heloderma* horridum, and exendin-4 [SEQ. ID. NO. 2] is present in the venom of *Heloderma suspectum* (Eng, J., et al., J. Biol. Chem., 265:20259-62, 1990; Eng., J., et al., J. Biol. Chem., 267:7402-05, 1992). The amino acid sequence of exendin-3 is shown in Figure 1. The amino acid sequence of exendin-4 is shown in Figure 2. The exendins have some sequence similarity to several members of the glucagon-like peptide family, with the highest homology, 53%, being to GLP-1[7-36]NH₂ [SEQ. ID. NO. 3] (Goke, et al., J. Biol. Chem., 268:19650-55, 1993). GLP-1[7-36]NH₂, also known as proglucagon[78-107] or simply "GLP-1" as used most often herein, has an insulinotropic effect, stimulating insulin secretion from pancreatic β-cells; GLP-1 also inhibits glucagon secretion from pancreatic α-cells (Ørsov, et al., Diabetes, 42:658-61, 1993; D'Alessio, et al., J. Clin. Invest., 97:133-38, 1996). The amino acid sequence of GLP-1 is shown in Figure 3. GLP-1 is reported to inhibit gastric emptying (Willms B, et al., J Clin Endocrinol Metab 81 (1): 327-32, 1996; Wettergren A, et al., Dig Dis Sci 38 (4): 665-73, 1993), and gastric acid secretion. Schjoldager BT, et al., Dig Dis Sci 34 (5): 703-8, 1989; O' Halloran DJ, et al., J Endocrinol 126 (1): 169-73, 1990; Wettergren A, et al., Dig Dis Sci 38 (4): 665-73, 1993). GLP-1[7-37], which has an additional glycine residue at its carboxy terminus, also stimulates insulin secretion in humans (Ørsov, et al., Diabetes, 42:658-61, 1993). A transmembrane G-protein adenylate-cyclase-coupled receptor believed to be responsible for the insulinotropic effect of GLP-1 has been cloned from a β-cell line (Thorens, Proc. Natl. Acad. Sci. USA 89:8641-45, 1992), hereinafter referred to as the "cloned GLP-1 receptor." Exendin-4 reportedly acts at GLP-1 receptors on insulin-secreting βTCl cells, at dispersed acinar cells from guinea pig pancreas, and at parietal cells from stomach; the peptide is also reported to stimulate somatostatin release and inhibit gastrin release in isolated stomachs (Goke, et al., J. Biol. Chem. 268:19650-55, 1993; Schepp, et al., Eur. J. Pharmacol., 69:183-91, 1994; Eissele, et al., Life Sci., 55:629-34, 1994). Exendin-3 and exendin-4 were reportedly found to stimulate cAMP production in, and amylase release from, pancreatic acinar cells (Malhotra, R., et al., Regulatory Peptides,41:149-56, 1992; Raufman, et al., J. Biol. Chem. 267:21432-37, 1992; Singh, et al., Regul. Pept. 53:47-59, 1994). Based on their insulinotropic activities, the use of exendin-3 and exendin-4 for the treatment of diabetes mellitus and the prevention of hyperglycemia has been proposed (Eng, U.S. Patent No. 5,424,286).

Agents which serve to delay gastric emptying have found a place in medicine as diagnostic aids in gastro-intestinal radiologic examinations. For example, glucagon is a polypeptide hormone which is produced by the α cells of the pancreatic islets of Langerhans. It is a hyperglycemic agent which mobilizes glucose by activating hepatic glycogenolysis. It can to a lesser extent stimulate the secretion of pancreatic insulin. Glucagon is used in the treatment of insulin-induced hypoglycemia, for example, when administration of glucose intravenously is not possible. However, as glucagon reduces the motility of the gastro-intestinal tract it is also used as a diagnostic aid in gastro-intestinal radiological examinations. Glucagon has also been used in several studies to treat various painful gastro-intestinal disorders associated with spasm. Daniel, et al. (Br. Med. J., 3:720, 1974) reported quicker symptomatic relief of acute diverticulitis in patients treated with glucagon compared with those who had been treated with analgesics or antispasmodics. A review by Glauser, et al. (J. Am. Coll. Emergency Physns, 8:228, 1979) described relief of acute esophageal food obstruction following glucagon therapy. In another study, glucagon significantly relieved pain and tenderness in 21 patients with biliary tract disease compared with 22 patients treated with placebo (M.J. Stower, et al., Br. J. Surg., 69:591-2, 1982).

Methods for regulating gastrointestinal motility using amylin agonists are described in International Application No. WO95/07098 published March 16, 1995.

Methods for regulating gastrointestinal motility using exendin agonists are described in U.S. 6,858,576 filed August 8, 1997 entitled "Methods for Regulating Gastrointestinal Motility.

Methods for reducing food intake using exendin agonists are described in U.S. described in U.S. 6,956,026 filed January 7, 1998, entitled "Use of Exendin and Agonists Thereof for the Reduction of Food Intake."

Novel exendin agonist compounds are described in PCT Application WO 99/07404 filed August 6, 1998, entitled "Novel Exendin Agonist Compounds," August 8, 1997. Other novel exendin agonists are described in Application No. WO 99/25727 filed November 13, 1998, entitled "Novel Exendin Agonist Compounds".

### SUMMARY OF THE INVENTION

According to one aspect the present invention provides a peptide compound of the formula [I] [SEQ. ID. NO. 4] wherein said peptide compound exhibits exendin agonist activity: wherein
Xaa₁ is His, Arg, Tyr, Ala, Norval, Val or Norleu;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys ;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
Xaa₂₇ is Ala or Lys;
Xaa₂₈ is Ala or Asn; and
Z₁ is Gly Gly -Z₂
   Gly Gly Xaa₃₁ - Z₂,
   Gly Gly Xaa₃₁ Ser -Z₂,
   Gly Gly Xaa₃₁ Ser Ser -Z₂,
   Gly Gly Xaa₃₁ Ser Ser Gly -Z₂,
   Gly Gly Xaa₃₁ Ser Ser Gly Ala -Z₂,
   Gly Gly Xaa₃₁ Ser Ser Gly Ala Xaa₃₆ -Z₂,
   Gly Gly Xaa₃₁ Ser Ser Gly Ala Xaa₃₆ Xaa₃₇ -Z₂,
   Gly Gly Xaa₃₁ Ser Ser Gly Ala Xaa₃₆ Xaa₃₇ Xaa₃₈ - Z₂ or
   Gly Gly Xaa₃₁ Ser Ser Gly Ala Xaa₃₆ Xaa₃₇ Xaa₃₈ Xaa₃₉ - Z₂;
   wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala; and pharmaceutically acceptable salts thereof; and wherein Xaa₁, Xaa₃ or Xaa₉ is Ala.

The present invention further provides the use of a respective compound for the manufacture of a pharmaceutical composition for the treatment of diabetes mellitus or the treatment of a hyperglycemic condition in a mammal.

In a further embodiment the present invention relates to a peptide compound of the formula (II) [SEQ. ID. NO. 94] wherein said peptide compound exhibits exendin agonist activity: wherein
Xaa₁ is His, Arg, Tyr, Ala, Norval, Val, Norleu or 4-imidazopropionyl;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala, Leu, or Lys- NH^{ε}-R where R is Lys, Arg, C₁-C₁₀ straight chain or branched alkanoyl or cycloalkylalkanoyl; Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
X₁ is Lys Asn, Asn Lys, Lys- NH^{ε}-R Asn, Asn Lys- NH^{ε}-R, Lys- NH^{ε}-R Ala, Ala Lys- NH^{ε}-R where R is Lys, Arg, C₁-C₁₀ straight chain or branched alkanoyl or cycloalkylalkanoyl; and
Z₁ is Gly Gly -Z₂,
   Gly Gly Xaa₃₁ -Z₂,
   Gly Gly Xaa₃₁ Ser -Z₂,
   Gly Gly Xaa₃₁ Ser Ser -Z₂,
   Gly Gly Xaa₃₁ Ser Ser Gly -Z₂,
   Gly Gly Xaa₃₁ Ser Ser Gly Ala -Z₂,
   Gly Gly Xaa₃₁ Ser Ser Gly Ala Xaa₃₆ -Z₂,
   Gly Gly Xaa₃₁ Ser Ser Gly Ala Xaa₃₆ Xaa₃₇ - -Z₂, or
   Gly Gly Xaa₃₁ Ser Ser Gly Ala Xaa₃₆ Xaa₃₇ Xaa₃₈ -Z₂;
   Gly Gly Xaa₃₁ Ser Ser Gly Ala Xaa₃₆ Xaa₃₇ Xaa₃₈ Xaa₃₉-Z₂;
   wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala;

In addition, the following abbreviations stand for the following :
"Norval" refers to norvaline
"Norleu" refers to norleucine

### BRIEF DESCRIPTION OF THE DRAWINGS

Figure 1 depicts the effect on lowering blood glucose of various concentrations of Compound 1 [SEQ. ID. NO. 5].

Figure 2 depicts a comparison of effects on gastric emptying of various concentrations of Compound 1 [SEQ. ID. NO. 5].

### DETAILED DESCRIPTION OF THE INVENTION

According to the present invention, provided are compounds of the formula (I) [SEQ. ID. NO. 4]:
wherein said peptide compound exhibits exendin agonist activity: wherein
Xaa₁ is His, Arg, Tyr, Ala, Norval, Val or Norleu;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
Xaa₂₇ is Ala or Lys;
Xaa₂₈ is Ala or Asn; and
Z₁ is Gly Gly -Z₂
   Gly Gly Xaa₃₁ -Z₂,
   Gly Gly Xaa₃₁ Ser -Z₂,
   Gly Gly Xaa₃₁ Ser Ser -Z₂,
   Gly Gly Xaa₃₁ Ser Ser Gly Z₂,
   Gly Gly Xaa₃₁ Ser Ser Gly Ala -Z₂,
   Gly Gly Xaa₃₁ Ser Ser Gly Ala Xaa₃₆ -Z₂,
   Gly Gly Xaa₃₁ Ser Ser Gly Ala Xaa₃₆ Xaa₃₇ -Z₂,
   Gly Gly Xaa₃₁ Ser Ser Gly Ala Xaa₃₆ Xaa₃₇ Xaa₃₈ -Z₂ or
   Gly Gly Xaa₃₁ Ser Ser Gly Ala Xaa₃₆ Xaa₃₇ Xaa₃₈ Xaa₃₉ - Z₂;
   wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, xaa₉, Xaa₁₀, Xaa₁₁, Xaa₁₂, Xaa₁₃ , Xaa₁₄, Xaa₁₅, Xaa₁₆, Xaa₁₇, Xaa₁₉, Xaa₂₀, Xaa₂₁, xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala; and pharmaceutically acceptable salts thereof; and wherein Xaa₁, Xaa₃ or Xaa₉ is Ala.

Within the compounds defined by the formula:
Xaa₂ can be Gly,
Xaa₄ can be Ala,
Xaa₁₄ can be Leu, pentylglycine or Met,
Xaa₂₅ can be Trp or Phe,
Xaa₆ can be Ala, Phe or naphthylalanine,
Xaa₂₂ can be Phe or naphthylalanine,
Xaa₂₃ can be Ile or Val,
Xaa₃₁, Xaa₃₆, Xaa₃₇ and Xaa₃₈ may independently be selected from the group consisting of Pro, homoproline, thioproline and N-alkylalanine,
Xaa₃₉ can be Ser,
Z_{2c} can be -NH₂.

Further embodiments of the present invention are represented by the compounds which have an amino acid sequence selected from SEQ. ID. NOS. 72, 73, 75-78, 80-83, 86, 88-90 and 92-93.

The compounds may further be present as a composition comprising a compound as defined above in a pharmaceutically acceptable carrier.

In a further aspect the present invention relates to the use of a compound according to claim 1 or 10 for the manufacture of a pharmaceutical composition for the treatment of diabetes mellitus. The composition may further comprise a therapeutically effective amount of an insulin.
According to a further aspect the present invention relates to the use of one of the above compounds for the manufacture of a pharmaceutical composition for the treatment of a hyperglycemic condition in a mammal.

In an alternative embodiment, the present invention is directed to a peptide compound of the formula (II) [SEQ. ID. NO. 94] wherein said peptide compound exhibits exendin agonist activity: wherein
Xaa₁ is His, Arg, Tyr, Ala, Norval, Val, Norleu or 4-imidazopropionyl;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala, Leu, or Lys- NH^{ε}-R where R is Lys, Arg, C₁-C₁₀ straight chain or branched alkanoyl or cycloalkylalkanoyl;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
X₁ is Lys Asn, Asn Lys, Lys- NH^{ε}-R Asn, Asn Lys- NH^{ε}-R, Lys- NH^{ε}-R Ala, Ala Lys- NH^{ε}-R where R is Lys, Arg, C₁-C₁₀ straight chain or branched alkanoyl or cycloalkylalkanoyl; and
Z₁ is Gly Gly -Z₂,
   Gly Gly Xaa₃₁ -Z₂,
   Gly Gly Xaa₃₁ Ser -Z₂,
   Gly Gly Xaa₃₁ Ser Ser -Z₂,
   Gly Gly Xaa₃₁ Ser Ser Gly -Z₂,
   Gly Gly Xaa₃₁ Ser Ser Gly Ala -Z₂,
   Gly Gly Xaa₃₁ Ser Ser Gly Ala Xaa₃₆ -Z₂,
   Gly Gly Xaa₃₁ Ser Ser Gly Ala Xaa₃₆ Xaa₃₇ -Z₂, or
   Gly Gly Xaa₃₁ Ser Ser Gly Ala Xaa₃₆ Xaa₃₇ Xaa₃₈ -Z₂;
   Gly Gly Xaa₃₁ Ser Ser Gly Ala Xaa₃₆ Xaa₃₇ Xaa₃₈ Xaa₃₉-Z₂;
   wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala; and pharmaceutically acceptable salts thereof; and wherein Xaa₁, Xaa₃ or Xaa₉ is Ala.

Within the compounds defined by the formula:
Xaa₂ can be Gly,
Xaa₄ can be Ala,
Xaa₁₄ can be Leu, pentylglycine or Met,
Xaa₂₅ can be Trp or Phe,
Xaa₆ can be Ala, Phe or naphthylalanine,
Xaa₂₂ can be Phe or naphthylalanine,
Xaa₂₃ can be Ile or Val,
Xaa₃₁, Xaa₃₆, Xaa₃₇ and Xaa₃₈ may be independently selected from the group consisting of Pro, homoproline, thioproline and N-alkylalanine,
Xaa₃₉ can be Ser,
Z₂ can be -NH₂.

In a further embodiment the invention is directed to one of these compounds, wherein X₁ can be Lys, Asn, Lys-NH^{ε}-R Asn, or Lys-NH^{ε}-R, Ala, R can be Lys, Arg, C₁-C₁₀ straight chain or branched alkanoyl.

Further compounds encompassed by the claims are characterized by the above formula, wherein Xaa₂₁ is Lys-NH^{ε}-R, where R is Lys, Arg, C₁-C₁₀ straight chain or branched alkanoyl or cycloalkyl-alkanoyl.

The compounds may for example have an amino acid sequence selected from SEQ. ID. NOS. 105, 106, 103, and 110.

The compounds may further be present as a composition comprising one of the above compounds in a pharmaceutically acceptable carrier.

The compounds referenced above form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include ammonium salts, alkali metal salts, e.g. sodium and potassium salts, and alkali earth salts, e.g. calcium and magnesium salts. Acetate, hydrochloride, and trifluoroacetate salts are preferred. The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

### Utility

The compounds described above are useful in view of their pharmacological properties. In particular, the compounds of the invention are exendin agonists, and possess activity as agents to regulate gastric motility and to slow gastric emptying, as evidenced by the ability to reduce post-prandial glucose levels in mammals.

The compounds of the present invention are useful in in vitro and in vivo scientific methods for investigation of exendins and exendin agonists for example in methods such as those described in Examples A-E below.

### Preparation of Compounds

The compounds of the present invention may be prepared using standard solid-phase peptide synthesis techniques and preferably an automated or semiautomated peptide synthesizer. Typically, using such techniques, an α-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain.

Suitable N-protecting groups are well known in the art, with t-butyloxycarbonyl (tBoc) and fluorenylmethoxycarbonyl (Fmoc) being preferred herein.

The solvents, amino acid derivatives and 4-methylbenzhydryl-amine resin used in the peptide synthesizer may be purchased from Applied Biosystems Inc. (Foster City, CA). The following side-chain protected amino acids may be purchased from Applied Biosystems, Inc.: Boc-Arg(Mts), Fmoc-Arg(Pmc), Boc-Thr(Bzl), Fmoc-Thr(t-Bu), Boc-Ser(Bzl), Fmoc-Ser(t-Bu), Boc-Tyr(BrZ), Fmoc-Tyr(t-Bu), Boc-Lys(C1-Z), Fmoc-Lys(Boc), Boc-Glu(Bzl), Fmoc-Glu(t-Bu), Fmoc-His(Trt), Fmoc-Asn(Trt), and Fmoc-Gln(Trt). Boc-His(BOM) may be purchased from Applied Biosystems, Inc. or Bachem Inc. (Torrance, CA). Anisole, dimethylsulfide, phenol, ethanedithiol, and thioanisole may be obtained from Aldrich Chemical Company (Milwaukee, WI). Air Products and Chemicals (Allentown, PA) supplies HF. Ethyl ether, acetic acid and methanol may be purchased from Fisher Scientific (Pittsburgh, PA).

Solid phase peptide synthesis may be carried out with an automatic peptide synthesizer (Model 430A, Applied Biosystems Inc., Foster City, CA) using the NMP/HOBt (Option 1) system and tBoc or Fmoc chemistry (see, Applied Biosystems User's Manual for the ABI 430A Peptide Synthesizer, Version 1.3B July 1, 1988, section 6, pp. 49-70, Applied Biosystems, Inc., Foster City, CA) with capping. Boc-peptide-resins may be cleaved with HF (-5°C to 0°C, 1 hour). The peptide may be extracted from the resin with alternating water and acetic acid, and the filtrates lyophilized. The Fmoc-peptide resins may be cleaved according to standard methods (Introduction to Cleavage Techniques, Applied Biosystems, Inc., 1990, pp. 6-12). Peptides may be also be assembled using an Advanced Chem Tech Synthesizer (Model MPS 350, Louisville, Kentucky).

Peptides may be purified by RP-HPLC (preparative and analytical) using a Waters Delta Prep 3000 system. A C4, C8 or C18 preparative column (10 µ, 2.2 x 25 cm; Vydac, Hesperia, CA) may be used to isolate peptides, and purity may be determined using a C4, C8 or C18 analytical column (5 µ, 0.46 x 25 cm; Vydac). Solvents (A=0.1% TFA/water and B=0.1% TFA/CH₃CN) may be delivered to the analytical column at a flowrate of 1.0 ml/min and to the preparative column at 15 ml/min. Amino acid analyses may be performed on the Waters Pico Tag system and processed using the Maxima program. Peptides may be hydrolyzed by vapor-phase acid hydrolysis (115°C, 20-24 h). Hydrolysates may be derivatized and analyzed by standard methods (Cohen, et al., The Pico Tag Method: A Manual of Advanced Techniques for Amino Acid Analysis, pp. 11-52, Millipore Corporation, Milford, MA (1989)). Fast atom bombardment analysis may be carried out by M-Scan, Incorporated (West Chester, PA). Mass calibration may be performed using cesium iodide or cesium iodide/glycerol. Plasma desorption ionization analysis using time of flight detection may be carried out on an Applied Biosystems Bio-Ion 20 mass spectrometer. Electrospray mass spectroscopy may be carried and on a VG-Trio machine.

Peptide compounds useful in the invention may also be prepared using recombinant DNA techniques, using methods now known in the art. See, e.g., Sambrook et al., Molecular Cloning: A Laboratory Manual, 2d Ed., Cold Spring Harbor (1989). Non-peptide compounds useful in the present invention may be prepared by art-known methods.

### Formulation and Administration

Compounds of the invention are useful in view of their exendin-like effects, and may conveniently be provided in the form of formulations suitable for parenteral (including intravenous, intramuscular and subcutaneous) or nasal, buccal or oral administration. In some cases, it will be convenient to provide an exendin agonist and another anti-gastric-emptying agent, such as glucagon, an amylin, or an amylin agonist, in a single composition or solution for administration together. In other cases, it may be more advantageous to administer another anti-emptying agent separately from said exendin agonist. In yet other cases, it may be beneficial to provide an exendin agonist either co-formulated or separately with other glucose lowering agents such as insulin. A suitable administration format may best be determined by a medical practitioner for each patient individually. Suitable pharmaceutically acceptable carriers and their formulation are described in standard formulation treatises, e.g., Remington's Pharmaceutical Sciences by E.W. Martin. See also Wang, Y.J. and Hanson, M.A. "Parenteral Formulations of Proteins and Peptides: Stability and Stabilizers," Journal of Parenteral Science and Technology, Technical Report No. 10, Supp. 42:2S (1988).

Compounds useful in the invention can be provided as parenteral compositions for injection or infusion. They can, for example, be suspended in an inert oil, suitably a vegetable oil such as sesame, peanut, olive oil, or other acceptable carrier. Preferably, they are suspended in an aqueous carrier, for example, in an isotonic buffer solution at a pH of about 5.6 to 7.4. These compositions may be sterilized by conventional sterilization techniques, or may be sterile filtered. The compositions may contain pharmaceutically acceptable auxiliary substances as required to approximate physiological conditions, such as pH buffering agents. Useful buffers include for example, sodium acetate/acetic acid buffers. A form of repository or "depot" slow release preparation may be used so that therapeutically effective amounts of the preparation are delivered into the bloodstream over many hours or days following transdermal injection or other form of delivery.

The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is preferred particularly for buffers containing sodium ions.

The claimed compounds can also be formulated as pharmaceutically acceptable salts (e.g., acid addition salts) and/or complexes thereof. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal administration and increasing the solubility to facilitate the administration of higher concentrations of the drug.

Pharmaceutically acceptable salts include acid addition salts such as those containing sulfate, hydrochloride, phosphate, sulfamate, acetate, citrate, lactate, tartrate, methanesulfonate, ethanesulfonate, benzenesulfonate, *p*-toluenesulfonate, cyclohexylsulfamate and quinate. Pharmaceutically acceptable salts can be obtained from acids such as hydrochloric acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

Carriers or excipients can also be used to facilitate administration of the compound. Examples of carriers and excipients include calcium carbonate, calcium phosphate, various sugars such as lactose, glucose, or sucrose, or types of starch, cellulose derivatives, gelatin, vegetable oils, polyethylene glycols and physiologically compatible solvents. The compositions or pharmaceutical composition can be administered by different routes including intravenously, intraperitoneal, subcutaneous, and intramuscular, orally, topically, or transmucosally.

If desired, solutions of the above compositions may be thickened with a thickening agent such as methyl cellulose. They may be prepared in emulsified form, either water in oil or oil in water. Any of a wide variety of pharmaceutically acceptable emulsifying agents may be employed including, for example, acacia powder, a non-ionic surfactant (such as a Tween), or an ionic surfactant (such as alkali polyether alcohol sulfates or sulfonates, e.g., a Triton).

Compositions useful in the invention are prepared by mixing the ingredients following generally accepted procedures. For example, the selected components may be simply mixed in a blender or other standard device to produce a concentrated mixture which may then be adjusted to the final concentration and viscosity by the addition of water or thickening agent and possibly a buffer to control pH or an additional solute to control tonicity.

For use by the physician, the compounds will be provided in dosage unit form containing an amount of an exendin agonist, with or without another anti-emptying agent. Therapeutically effective amounts of an exendin agonist for use in the control of gastric emptying and in conditions in which gastric emptying is beneficially slowed or regulated are those that decrease post-prandial blood glucose levels, preferably to no more than about 8 or 9 mM or such that blood glucose levels are reduced as desired. In diabetic or glucose intolerant individuals, plasma glucose levels are higher than in normal individuals. In such individuals, beneficial reduction or "smoothing" of post-prandial blood glucose levels, may be obtained. As will be recognized by those in the field, an effective amount of therapeutic agent will vary with many factors including the patient's physical condition, the blood sugar level or level of inhibition of gastric emptying to be obtained, and other factors.

Such pharmaceutical compositions are useful in causing gastric hypomotility in a subject and may be used as well in other disorders where gastric motility is beneficially reduced.

The effective daily anti-emptying dose of the compounds will typically be in the range of 0.001 or 0.005 to about 5 mg/day, preferably about 0.01 or 0.05 to 2 mg/day and more preferably about 0.05 or 0.1 to 1 mg/day, for a 70 kg patient. The exact dose to be administered is determined by the attending clinician and is dependent upon where the particular compound lies within the above quoted range, as well as upon the age, weight and condition of the individual. Administration should begin at the first sign of symptoms or shortly after diagnosis of diabetes mellitus. Administration may be by injection, preferably subcutaneous or intramuscular. Administration may also be by other routes, for example, by oral, buccal or nasal routes, however dosages should be increased about 5-10 fold, over injection doses.

Generally, in treating or preventing elevated, inappropriate, or undesired post-prandial blood glucose levels, the compounds of this invention may be administered to patients in need of such treatment in a dosage ranges similar to those given above, however, the compounds are administered more frequently, for example, one, two, or three times a day.

The optimal formulation and mode of administration of compounds of the present application to a patient depend on factors known in the art such as the particular disease or disorder, the desired effect, and the type of patient. While the compounds will typically be used to treat human patients, they may also be used to treat similar or identical diseases in other vertebrates such as other primates, farm animals such as swine, cattle and poultry, and sports animals and pets such as horses, dogs and cats.

To assist in understanding the present invention the following Examples are included which describe the results of a series of experiments. The experiments relating to this invention should not, of course, be construed as specifically limiting the invention and such variations of the invention, now known or later developed, which would be within the purview of one skilled in the art are considered to fall within the scope of the invention as described herein and hereinafter claimed.

### EXAMPLE 1

### Preparation of Compound 68

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 4033.5.

### EXAMPLE 2

### Preparation of Compound 69

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3984.4.

### EXAMPLE 3

### Preparation of Compound 71

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3861.3.

### EXAMPLE 4

### Preparation of Compound 72

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3746.1.

### EXAMPLE 5

### Preparation of Compound 73

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3742.1.

### EXAMPLE 6

### Preparation of Compound 74

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3693.1.

### EXAMPLE 7

### Preparation of Compound 76

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3634.1.

### EXAMPLE 8

### Preparation of Compound 77

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3526.9.

### EXAMPLE 9

### Preparation of Compound 78

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3477.9.

### EXAMPLE 10

### Preparation of Compound 79

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3519.9.

### EXAMPLE 11

### Preparation of Compound 82

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Double couplings are required at residues 37,36 and 31. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 4121.1.

### EXAMPLE 12

### Preparation of Compound 84

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Double couplings are required at residues 36 and 31. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3796.1.

### EXAMPLE 13

### Preparation of Compound 85

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. A double coupling is required at residue 31. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3871.1.

### EXAMPLE 14

### Preparation of Compound 86

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino-acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3750.2.

### EXAMPLE 15

### Preparation of Compound 88

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 4120.6.

### EXAMPLE 16

### Preparation of Compound 89

The above-identified amidated peptide is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Compound 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 4005.5.

### EXAMPLE 17

### Preparation of Peptide having SEO. ID. NO. 103

Compound No. 98, Ala Gly Glu Gly Thr Phe Thr Ser Asp Leu Ser Lys Gln Met Glu Glu Glu Ala Val Arg Leu Phe Ile Glu Trp Leu Lys-NH^{ε}octanoyl Asn-*NH₂* [SEQ. ID. NO. 103], is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Fmoc-Lys-NH^{ε}octanoyl acid is used for coupling at position 27. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3334.6

### EXAMPLE 18

### Preparation of Peptide having SEQ. ID. NO. 105

Compound No. 100, Ala Gly Glu Gly Thr Phe Thr Ser Asp Leu Ser Lys Gln Met Glu Glu Glu Ala Val Arg Leu Phe Ile Glu Trp Leu Lys-NH^{ε}octanoyl Asn Gly Gly-*NH₂* [SEQ. ID. NO. 105], is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Fmoc-Lys-NH^{ε}octanoyl acid is used for coupling at position 27. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3442.8

### EXAMPLE 19

### Preparation of Peptide having SEQ ID. NO. 106

Compound No. 101, Ala Gly Glu Gly Thr Phe Thr Ser Asp Leu Ser Lys Gln Leu Glu Glu Glu Ala Val Arg Leu Phe Ile Glu Phe Leu Lys-NH^{ε}octanoyl Asn Gly Gly-*NH*₂ [SEQ. ID. NO. 106], is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Fmoc-Lys-NH^{ε}octanoyl acid is used for coupling at position 27. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3391.7

### EXAMPLE 20

### Preparation of Peptide having SEO. ID. NO. 110

Compound No. 105, Ala Gly Glu Gly Thr Phe Thr Ser Asp Leu Ser Lys Gln Leu Glu Glu Glu Ala Val Arg Leu Phe Ile Glu Phe Leu Asn Lys-NH^{ε}octanoyl Gly Gly-*NH*₂ [SEQ. ID. NO. 110], is assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Fmoc-Lys-NH^{ε}octanoyl acid is used for coupling at position 28. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry (M): calculated 3391.7

### EXAMPLES A TO E

### Reagents Used

GLP-1[7-36]NH₂ (GLP-1) was purchased from Bachem (Torrance, CA). All other peptides were prepared using synthesis methods such as those described therein. All chemicals were of the highest commercial grade. The cAMP SPA immunoassay was purchased from Amersham. The radioligands were purchased from New England Nuclear (Boston, MA). RINm5f cells (American Type Tissue Collection, Rockville, MD) were grown in DME/F12 medium containing 10% fetal bovine serum and 2mM L-glutamine. Cells were grown at 37°C and 5% CO₂/95% humidified air and medium was replaced every 2 to 3 days. Cells were grown to confluence then harvested and homogenized using on a Polytron homogenizer. Cell homogenates were stored frozen at -70°C until used.

### EXAMPLE A

### GLP-1 Receptor Binding Studies

Receptor binding was assessed by measuring displacement of [²⁵¹]GLP-1 or [¹²⁵I]exendin(9-39) from RINm5f membranes. Assay buffer contained 5 µg/ml bestatin, 1 µg/ml phosphoramidon, 1 mg/ml bovine serum albumin (fraction V), 1 mg/ml bacitracin, and 1 mM MgCl₂ in 20 mM HEPES, pH 7.4. To measure binding, 30 µg membrane protein (Bradford protein assay) was resuspended in 200 µl assay buffer and incubated with 60 pM [¹²⁵I]GLP-1 or [¹²⁵I]exendin(9-39) and unlabeled peptides for 120 minutes at 23°C in 96 well plates (Nagle Nunc, Rochester, NY). Incubations were terminated by rapid filtration with cold phosphatebuffered saline, pH 7.4, through polyethyleneimine-treated GF/B glass fiber filters (Wallac Inc., Gaithersburg, MD) using a Tomtec Mach II plate harvester (Wallac Inc., Gaithersburg, MD). Filters were dried, combined with scintillant, and radioactivity determined in a Betaplate liquid scintillant counter (Wallac Inc.).

Peptide samples were run in the assay as duplicate points at 6 dilutions over a concentration range of 10⁻⁶M to 10⁻¹²M to generate response curves. The biological activity of a sample is expressed as an IC₅₀ value, calculated from the raw data using an iterative curve-fitting program using a 4-parameter logistic equation (Prizm^{™}, GraphPAD Software). The results are shown in Table I.

**TABLE I**

| Compound | IC₅₀ (nM) |
|---|---|
| Exendin-4 [SEQ. ID. NO. 2] | 0.7 |
| Compound 1 [SEQ. ID. NO. 5] | 26.1 |
| Compound 2 [SEQ. ID. NO. 6] | 14.42 |
| Compound 3 [SEQ. ID. NO. 7] | 41.65 |
| Compound 4 [SEQ. ID. NO. 8] | 4.96 |

### EXAMPLE B

### Cyclase Activation Study

Assay buffer contained 10 µM GTP, 0.75 mM ATP, 2.5 mM MgCl₂, 0.5mM phosphocreatine, 12.5 U/ml creatine kinase, 0.4 mg/ml aprotinin, 1 µM IBMX in 50 mM HEPES, pH 7.4. Membranes and peptides were combined in 100 ml of assay buffer in 96 well filter-bottom plates (Millipore Corp., Bedford, MA). After 20 minutes incubation at 37°C, the assay was terminated by transfer of supernatant by filtration into a fresh 96 well plate using a Millipore vacuum manifold. Supernatant cAMP contents were quantitated by SPA immunoassay.

Peptide samples were run in the assay as triplicate points at 7 dilutions over a concentration range of 10⁻⁶M to 10⁻¹²M to generate response curves. The biological activity of a particular sample was expressed as an EC₅₀ value, calculated as described above. Results are tabulated in Table II.

**TABLE II**

| **Compound** | | EC₅₀(nM) |
|---|---|---|
| Exendin-4 | [SEQ. ID. NO. 2] | 0.23 |
| Compound 1 | [SEQ. ID. NO. 5] | >1,000 |
| Compound 2 | [SEQ. ID. NO. 6] | >10,000 |
| Compound 3 | [SEQ. ID. NO. 7] | >10,000 |
| Compound 4 | [SEQ. ID. NO. 8] | >10,000 |

### EXAMPLE C

### Determination of Blood Glucose Levels in db/db Mice

C57BLKS/J-m-db mice at least 3 months of age were utilized for the study. The mice were obtained from The Jackson Laboratory and allowed to acclimate for at least one week before use. Mice were housed in groups of ten at 22° ± 1°C with a 12:12 light:dark cycle, with lights on at 6 a.m. All animals were deprived of food for 2 hours before taking baseline blood samples. Approximately 70 µl of blood was drawn from each mouse via eye puncture, after a light anesthesia with metophane. After collecting baseline blood samples, to measure plasma glucose concentrations, all animals receive subcutaneous injections of either vehicle (10.9% NaCl), exendin-4 or test compound (1 µg) in vehicle. Blood samples were drawn again, using the same procedure, after exactly one hour from the injections, and plasma glucose concentrations were measured.

For each animal, the % change in plasma value, from baseline value, was calculated. The percent decrease in plama glucose after one hour is shown in Table III.

**TABLE III**

| Test Compound | % drop in glucose | |
|---|---|---|
| Exendin-4 [SEQ. ID. NO. 2] | 39% | (n = 78) |
| Compound 1 [SEQ. ID. NO. 5] | 40% | (n = 4) |
| Compound 2 [SEQ. ID. NO. 6] | 41% | (n = 5) |
| Compound 3 [SEQ. ID. NO.7] | 32% | (n = 5) |
| Compound 4 [SEQ. ID. NO. 8] | 42% | (n = 5) |

### EXAMPLE D

### Dose Response Determination of Blood Glucose Levels in db/db Mice

C57BLKS/J-m-db/db mice, at least 3 months of age were utilized for the study. The mice were obtained from The Jackson Laboratory and allowed to acclimate for at least one week before use. Mice were housed in groups of ten at 22°C 1°C with a 12:12 light:dark cycle, with lights on at 6 a.m.

All animals were deprived of food for 2 hours before taking baseline blood samples. Approximately 70 µl of blood was drawn from each mouse via eye puncture, after a light anesthesia with metophane. After collecting baseline blood samples, to measure plasma glucose concentrations, all animals receive subcutaneous injections of either vehicle, exendin-4 or test compound in concentrations indicated. Blood samples were drawn again, using the same procedure, after exactly one hour from the injections, and plasma glucose concentrations were measured.

For each animal, the % change in plasma value, from baseline value, was calculated and a dose dependent relationship was evaluated using Graphpad Prizm^{™} software.

Figure 1 depicts the effects of varying doses of exendin-4 [SEQ. ID. NO. 2] and Compound 1 [SEQ. ID. NO. 5] on plasma glucose levels. Exendin-4 had an ED₅₀ of 0.01 µg per mouse and Compound 1 had an ED₅₀ of 0.42 µg per mouse.

### EXAMPLE E

### Gastric Emptying

The following study was carried out to examine the effects of exendin-4 and an exendin agonist compound of the present invention on gastric emptying in rats. This experiment followed a modification of the method of Scarpignato, et al., Arch. Int. Pharmacodyn. Ther. 246:286-94, 1980.

Male Harlan Sprague Dawley (HSD) rats were used. All animals were housed at 22.7 ± 0.8 C in a 12:12 hour light:dark cycle (experiments being performed during the light cycle) and were fed and watered *ad libitum* (Diet LM-485, Teklad, Madison, WI). Exendin-4 was synthesized according to standard peptide synthesis methods. The preparation of Compound 1 [SEQ. ID. NO. 5] is described in Example 1.

The determination of gastric emptying by the method described below was performed after a fast of -20 hours to ensure that the stomach contained no chyme that would interfere with spectrophotometric absorbance measurements.

Conscious rats received by gavage, 1.5ml of an acaloric gel containing 1.5% methyl cellulose (M-0262, Sigma Chemical Co, St Louis, MO) and 0.05% phenol red indicator. Twenty minutes after gavage, rats were anesthetized using 5% halothane, the stomach exposed and clamped at the pyloric and lower esophageal sphincters using artery forceps, removed and opened into an alkaline solution which was made up to a fixed volume. Stomach content was derived from the intensity of the phenol red in the alkaline solution, measured by absorbance at a wavelength of 560 nm. In separate experiments on 7 rats, the stomach and small intestine were both excised and opened into an alkaline solution. The quantity of phenol red that could be recovered from the upper gastrointestinal tract within 20 minutes of gavage was 89±4%; dye which appeared to bind irrecoverably to the gut luminal surface may have accounted for the balance. To account for a maximal dye recovery of less than 100%, percent of stomach contents remaining after 20 min were expressed as a fraction of the gastric contents recovered from control rats sacrificed immediately after gavage in the same experiment. Percent gastric contents remaining = (absorbance at 20 min)/(absorbance at 0 mm) x 100.

In baseline studies, with no drug treatment, gastric emptying over 20 min was determined. In dose-response studies, rats were treated with 0.01, 0.1, 0.3, 1, 10 and 100 µg of exendin-4, and 0.1, 0.3, 1, 10 and 100 µg of Compound 1 [SEQ. ID. NO. 5].

The results, shown in Figure 2, demonstrate that the exendin agonists, exendin-4 and Compound 1, are potent inhibitors of gastric emptying. The EC₅₀ for exendin-4 was 0.27 µg. The EC₅₀ for Compound 1 was 55.9 µg.

## Claims

1. A peptide compound of the formula [I] [SEQ. ID. NO. 4] wherein said peptide compound exhibits exendin agonist activity: wherein
Xaa₁ is His, Arg, Tyr, Ala, Norval, Val or Norleu;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
Xaa₂₇ is Ala or Lys;
Xaa₂₈ is Ala or Asn; and
Z₁ is Gly Gly -Z₂
Gly Gly Xaa₃₁ -Z₂,
Gly Gly Xaa₃₁ Ser -Z₂,
Gly Gly Xaa₃₁ Ser Ser -Z₂,
Gly Gly Xaa₃₁ Ser Ser Gly -Z₂,
Gly Gly Xaa₃₁ Ser Ser Gly Ala -Z₂,
Gly Gly Xaa₃₁ Ser Ser Gly Ala Xaa₃₆ -Z₂,
Gly Gly Xaa₃₁ Ser Ser Gly Ala Xaa₃₆ Xaa₃₇ -Z₂,
Gly Gly Xaa₃₁ Ser Ser Gly Ala Xaa₃₆ Xaa₃₇ Xaa₃₈ -Z₂ or Gly Gly Xaa₃₁ Ser Ser Gly Ala Xaa₃₆ Xaa₃₇ Xaa₃₈ Xaa₃₉ - Z₂;
wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala; and pharmaceutically acceptable salts thereof; and wherein Xaa₁, Xaa₃ or Xaa₉ is Ala.

2. A compound according to claim 1 wherein Xaa₂ is Gly.

3. A compound according to claim 1 or 2 wherein Xaa₄ is Ala.

4. A compound according to claim 2 or 3 wherein Xaa₁₄ is Leu, pentylglycine or Met.

5. A compound according to claim 4 wherein Xaa₂₅ is Trp or Phe.

6. A compound according to claim 5 wherein Xaa₆ is Ala, Phe or naphthylalanine; Xaa₂₂ is Phe or naphthylalanine; and Xaa₂₃ is Ile or Val.

7. A compound according to claim 1 or 6 wherein Xaa₃₁, Xaa₃₆, Xaa₃₇ and Xaa₃₈ are independently selected from the group consisting of Pro, homoproline, thioproline and N-alkylalanine.

8. A compound according to claim 1 or 6 wherein Xaa₃₉ is Ser.

9. A compound according to any of claims 1, 7, or 8, wherein Z₂ is -NH₂.

10. A compound according to claim 1 which has an amino acid sequence selected from SEQ. ID. NOS. 72, 73, 75-78, 80-83, 86, 88-90 and 92-93.

11. A composition comprising a compound of any of claims 1 to 10 in a pharmaceutically acceptable carrier.

12. Use of a compound according to claim 1 or 10 for the manufacture of a pharmaceutical composition for the treatment of diabetes mellitus.

13. The use according to claim 12, said composition further comprising a therapeutically effective amount of an insulin.

14. Use of a compound according to claim 1 or 10 for the manufacture of a pharmaceutical composition for the treatment of a hyperglycemic condition in a mammal.

15. A peptide compound of the formula (II) [SEQ. ID. NO. 94] wherein said peptide compound exhibits exendin agonist activity: wherein
Xaa₁ is His, Arg, Tyr, Ala, Norval, Val, Norleu or 4-imidazopropionyl;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala, Leu, or Lys- NH^{ε}-R where R is Lys, Arg, C₁-C₁₀ straight chain or branched alkanoyl or cycloalkylalkanoyl; Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
X₁ is Lys Asn, Asn Lys, Lys- NH^{ε}-R Asn, Asn Lys- NH^{ε}-R,
Lys- NH^{ε}-R Ala, Ala Lys- NH^{ε}-R where R is Lys, Arg, C₁-C₁₀ straight chain or branched alkanoyl or cycloalkylalkanoyl; and
Z₁ is Gly Gly -Z₂,
Gly Gly Xaa₃₁ -Z₂,
Gly Gly Xaa₃₁ Ser -Z₂,
Gly Gly Xaa₃₁ Ser Ser -Z₂,
Gly Gly Xaa₃₁ Ser Ser Gly -Z₂,
Gly Gly Xaa₃₁ Ser Ser Gly Ala -Z₂,
Gly Gly Xaa₃₁ Ser Ser Gly Ala Xaa₃₆ -Z₂,
Gly Gly Xaa₃₁ Ser Ser Gly Ala Xaa₃₆ Xaa₃₇ -Z₂, or
Gly Gly Xaa₃₁ Ser Ser Gly Ala Xaa₃₆ Xaa₃₇ Xaa₃₈ -Z₂;
Gly Gly Xaa₃₁ Ser Ser Gly Ala Xaa₃₆ Xaa₃₇ Xaa₃₈ Xaa₃₉-Z₂;
wherein Xaa₃₁, Xaa₃₆, Xaa₃₇ and Xaa₃₈ are independently selected from the group consisting of Pro, homoproline, 3Hyp, 4Hyp, thioproline, N-alkylglycine, N-alkylpentylglycine and N-alkylalanine; Xaa₃₉ is Ser or Tyr; and Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala; and pharmaceutically acceptable salts thereof; and wherein Xaa₁, Xaa₃ or Xaa₉ is Ala.

16. A compound according to claim 15 wherein Xaa₂ is Gly.

17. A compound according to claim 15 wherein Xaa₄ is Ala.

18. A compound according to claim 15 wherein Xaa₁₄ is Leu, pentylglycine or Met.

19. A compound according to claim 15 wherein Xaa₂₅ is Trp or Phe.

20. A compound according to claim 15 wherein Xaa₆ is Ala, Phe or naphthylalanine; Xaa₂₂ is Phe or naphthylalanine; and Xaa₂₃ is Ile or Val.

21. A compound according to claim 15 wherein Xaa₃₁, Xaa₃₆, Xaa₃₇ and Xaa₃₈ are independently selected from the group consisting of Pro, homoproline, thioproline and N-alkylalanine.

22. A compound according to claim 15 or 21 where Xaa₃₉ is Ser.

23. A compound according to claim 15 wherein Z₂ is -NH₂.

24. A compound according to claim 15 wherein X₁ is Lys Asn, Lys-NH^{ε}-R Asn, or Lys-NH^{ε}-R Ala where R is Lys, Arg, C₁-C₁₀ straight chain or branched alkanoyl.

25. A compound according to claim 15 wherein Xaa₂₁ is Lys-NH^{ε}-R where R is Lys, Arg, C₁-C₁₀ straight chain or branched alkanoyl or cycloalkyl-alkanoyl.

26. A compound according to claim 15 which has an amino acid sequence selected from SEQ. ID. NOS. 105, 106, 103, and 110.

27. A composition comprising a compound of claim 15 or 26 in a pharmaceutically acceptable carrier.

## Patentansprüche

1. Peptidverbindung der Formel (I) [SEQ ID Nr:4], wobei die Peptidverbindung Exendin-Agonistenaktivität aufweist: wobei
Xaa₁ His, Arg, Tyr, Ala, Norval, Val oder Norleu ist;
Xaa₂ Ser, Gly, Ala oder Thr ist;
Xaa₃ Ala, Asp oder Glu ist;
Xaa₄ Ala, Norval, Val, Norleu oder Gly ist;
Xaa₅ Ala oder Thr ist;
Xaa₆ Phe, Tyr oder Naphthylalanin ist;
Xaa₇ Thr oder Ser ist;
Xaa₈ Ala, Ser oder Thr ist;
Xaa₉ Ala, Norval, Val, Norleu, Asp oder Glu ist;
Xaa₁₀ Ala, Leu, Ile, Val, Pentylglycin oder Met ist;
Xaa₁₁ Ala oder Ser ist;
Xaa₁₂ Ala oder Lys ist;
Xaa₁₃ Ala oder Gln ist;
Xaa₁₄ Ala, Leu, Ile, Pentylglycin, Val oder Met ist;
Xaa₁₅ Ala oder Glu ist;
Xaa₁₆ Ala oder Glu ist;
Xaa₁₇ Ala oder Glu ist;
Xaa₁₉ Ala oder Val ist;
Xaa₂₀ Ala oder Arg ist;
Xaa₂₁ Ala oder Leu ist;
Xaa₂₂ Phe, Tyr oder Naphthylalanin ist;
Xaa₂₃ Ile, Val, Leu, Pentylglycin, Tert-Butylglycin oder Met ist;
Xaa₂₄ Ala, Glu oder Asp ist;
Xaa₂₅ Ala, Trp, Phe, Tyr oder Naphthylalanin ist;
Xaa₂₆ Ala oder Leu ist;
Xaa₂₇ Ala oder Lys;
Xaa₂₈ Ala oder Asn ist; und wobei Xaa₃₁, Xaa₃₆, Xaa₃₇ und Xaa₃₈ unabhängig voneinander aus der Gruppe bestehend aus Pro, Homoprolin, 3Hyp, 4Hyp, Thioprolin, N-Alkylglycin, N-Alkylpentylglycin und N-alkylalanin ausgewählt sind; Xaa₃₉ Ser oder Tyr ist; und Z₂ -OH oder -NH₂ ist;
vorausgesetzt, daß nicht mehr als drei von Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ und Xaa₂₈ Ala sind; und ebenfalls vorausgesetzt, daß wenn Xaa₁ His, Arg oder Tyr ist, dann mindestens eins von Xaa₃, Xaa₄ und Xaa₉ Ala ist; und pharmazeutisch verträgliche Salze davon; und wobei Xaa₁, Xaa₃ oder Xaa₉ Ala ist.

2. Verbindung nach Anspruch 1, wobei Xaa₂ Gly ist.

3. Verbindung nach Anspruch 1 oder 2, wobei Xaa₄ Ala ist.

4. Verbindung nach Anspruch 2 oder 3, wobei Xaa₁₄ Leu, Pentylglycin oder Met ist.

5. Verbindung nach Anspruch 4, wobei Xaa₂₅ Trp oder Phe ist.

6. Verbindung nach Anspruch 5, wobei Xaa₆ Ala, Phe oder Naphtylalanin ist; Xaa₂₂ Phe oder Naphthylalanin ist; und Xaa₂₃ Ile oder Val ist.

7. Verbindung nach Anspruch 1 oder 6, wobei Xaa₃₁, Xaa₃₆, Xaa₃₇ und Xaa₃₈ unabhängig voneinander aus der Gruppe bestehend aus Pro, Homoprolin, Thioprolin und N-Alkylalanin ausgewählt sind.

8. Verbindung nach Anspruch 1 oder 6, wobei Xaa₃₉ Ser ist.

9. Verbindung nach einem der Ansprüche 1, 7 oder 8, wobei Z₂ -NH₂ ist.

10. Verbindung nach Anspruch 1, die eine Aminosäuresequenz ausgewählt aus SEQ ID Nr.: 72, 73, 75-78, 80-83, 86, 88-90 und 92-93 aufweist.

11. Zusammensetzung, die eine Verbindung gemäß mindestens einem der Ansprüche 1 bis 10 in einem pharmazeutisch verträglichen Träger umfaßt.

12. Verwendung einer Verbindung gemäß Anspruch 1 oder 10 für die Herstellung einer pharmazeutischen Zusammensetzung für die Behandlung von Diabetes mellitus.

13. Verwendung nach Anspruch 12, wobei die Zusammensetzung ferner eine therapeutisch wirksame Menge eines Insulins umfaßt.

14. Verwendung einer Verbindung gemäß Anspruch 1 oder 10 für die Herstellung einer pharmazeutischen Zusammensetzung für die Behandlung eines hyperglykämischen Zustandes in einem Säugetier.

15. Peptidverbindung der Formel (II) [SEQ ID Nr. 94], wobei die Peptidverbindung Exendin-Agonistenaktivität aufweist: wobei
Xaa₁ His, Arg, Tyr, Ala, Norval, Val, Norleu oder 4-Imidazopropionyl ist;
Xaa₂ Ser, Gly, Ala oder Thr ist;
Xaa₃ Ala, Asp oder Glu ist;
Xaa₄ Ala, Norval, Val, Norleu oder Gly ist;
Xaa₅ Ala oder Thr ist;
Xaa₆ Phe, Tyr oder Naphthylalanin ist;
Xaa₇ Thr oder Ser ist;
Xaa₈ Ala, Ser oder Thr ist;
Xaa₉ Ala, Norval, Val, Norleu, Asp oder Glu ist;
Xaa₁₀ Ala, Leu, Ile, Val, Pentylglycin oder Met ist;
Xaa₁₁ Ala oder Ser ist;
Xaa₁₂ Ala oder Lys ist;
Xaa₁₃ Ala oder Gln ist;
Xaa₁₄ Ala, Leu, Ile, Pentylglycin, Val oder Met ist;
Xaa₁₅ Ala oder Glu ist;
Xaa₁₆ Ala oder Glu ist;
Xaa₁₇ Ala oder Glu ist;
Xaa₁₉ Ala oder Val ist;
Xaa₂₀ Ala oder Arg ist;
Xaa₂₁ Ala, Leu oder Lys- NH^{ε}-R ist, wobei R Lys, Arg, C₁-C₁₀ geradkettiges oder verzweigtes Alkanoyl oder Cycloalkylalkanoyl ist;
Xaa₂₂ Phe, Tyr oder Naphthylalanin ist;
Xaa₂₃ Ile, Val, Leu, Pentylglycin, tert-Butylglycin oder Met ist;
Xaa₂₄ Ala, Glu oder Asp ist;
Xaa₂₅ Ala, Trp, Phe, Tyr oder Naphthylalanin ist;
Xaa₂₆ Ala oder Leu ist;
X₁ Lys Asn, Asn Lys, Lys- NH^{ε}-R Asn, Asn Lys- NH^{ε}-R, Lys- NH^{ε}-R Ala, Ala Lys- NH^{ε}-R ist, wobei R Lys, Arg, C₁-C₁₀ geradkettiges oder verzweigtes Alkanoyl oder Cycloalkylalkanoyl ist; und wobei Xaa₃₁, Xaa₃₆, Xaa₃₇ und Xaa₃₈ unabhängig voneinander aus der Gruppe bestehend aus Pro, Homoprolin, 3Hyp, 4Hyp, Thioprolin, N-Alkylglycin, N-Alkylpentylglycin und N-Alkylalanin ausgewählt sind; Xaa₃₉ Ser oder Tyr ist; und Z₂ -OH oder -NH₂ ist;
vorausgesetzt, daß nicht mehr als drei von Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ und Xaa₂₈ Ala sind; und ebenfalls vorausgesetzt, daß wenn Xaa₁ His, Arg oder Tyr ist, dann mindestens eins von Xaa₃, Xaa₄, und Xaa₉ Ala ist; und pharmazeutisch verträgliche Salze davon; und wobei Xaa₁, Xaa₃ oder Xaa₉ Ala ist.

16. Verbindung nach Anspruch 15, wobei Xaa₂ Gly ist.

17. Verbindung nach Anspruch 15, wobei Xaa₄ Ala ist.

18. Verbindung nach Anspruch 15, wobei Xaa₁₄ Leu, Pentylglycin oder Met ist.

19. Verbindung nach Anspruch 15, wobei Xaa₂₅ Trp oder Phe ist.

20. Verbindung nach Anspruch 15, wobei Xaa₆ Ala, Phe oder Naphthylalanin ist; Xaa₂₂ Phe oder Naphtylalanin ist; und Xaa₂₃ Ile oder Val ist.

21. Verbindung nach Anspruch 15, wobei Xaa₃₁, Xaa₃₆, Xaa₃₇ und Xaa₃₈ unabhängig voneinander aus der Gruppe bestehend aus Pro, Homoprolin, Thioprolin und N-Alkylalanin ausgewählt sind.

22. Verbindung nach Anspruch 15 oder 21, wobei Xaa₃₉ Ser ist.

23. Verbindung nach Anspruch 15, wobei Z₂ -NH₂ ist.

24. Verbindung nach Anspruch 15, wobei X₁ Lys Asn, Lys-NH^{ε}-R Asn oder Lys-NH^{ε}-R Ala ist, wobei R Lys, Arg, C₁-C₁₀ geradkettiges oder verzweigtes Alkanoyl ist.

25. Verbindung nach Anspruch 15, wobei Xaa₂₁ Lys-NH^{ε}-R ist, WObei R Lys, Arg, C₁-C₁₀ geradkettiges oder verzweigtes Alkanoyl oder Cycloalkyl-Alkanoyl ist.

26. Verbindung nach Anspruch 15, die eine Aminosäuresequenz ausgewählt aus SEQ ID Nrn.: 105, 106, 103 und 110 aufweist.

27. Zusammensetzung, die eine Verbindung gemäß Anspruch 15 oder 26 in einem pharmazeutisch verträglichen Träger umfaßt.

## Revendications

1. Composé peptidique de formule (I) (SEQ ID n° 4), dans lequel ledit composé peptidique présente une activité agoniste de l'exendine : dans lequel
Xaa₁ est His, Arg, Tyr, Ala, Norval, Val ou Norleu ;
Xaa₂ est Ser, Gly, Ala ou Thr ;
Xaa₃ est Ala, Asp ou Glu ;
Xaa₄ est Ala, Norval, Val, Norleu ou Gly ;
Xaa₅ est Ala ou Thr ;
Xaa₆ est Phe, Tyr ou la naphtylalanine ;
Xaa₇ est Thr ou Ser ;
Xaa₈ est Ala, Ser ou Thr ;
Xaa₉ est Ala, Norval, Val, Norleu, Asp ou Glu ;
Xaa₁₀ est Ala, Leu, Ile, Val, la pentylglycine ou Met ;
Xaa₁₁ est Ala ou Ser ;
Xaa₁₂ est Ala ou Lys ;
Xaa₁₃ est Ala ou Gln ;
Xaa₁₄ est Ala, Leu, Ile, la pentylglycine, Val ou Met ;
Xaa₁₅ est Ala ou Glu ;
Xaa₁₆ est Ala ou Glu ;
Xaa₁₇ est Ala ou Glu ;
Xaa₁₉ est Ala ou Val ;
Xaa₂₀ est Ala ou Arg ;
Xaa₂₁ est Ala ou Leu ;
Xaa₂₂ est Phe, Tyr ou la naphtylalanine ;
Xaa₂₃ est Ile, Val, Leu, la pentylglycine, la tert-butylglycine ou Met;
Xaa₂₄ est Ala, Glu ou Asp ;
Xaa₂₅ est Ala, Trp, Phe, Tyr ou la naphtylalanine ; Xaa₂₆ est Ala ou Leu ;
Xaa₂₇ est Ala ou Lys ;
Xaa₂₈ est Ala ou Asn ; et dans lesquelles Xaa₃₁, Xaa₃₆, Xaa₃₇ et Xaa₃₈ sont choisis indépendamment dans le groupe constitué par Pro, l'homoproline, 3Hyp, 4Hyp, la thioproline, une N-alkylglycine, une N-alkylpentylglycine et une N-alkylalanine ; Xaa₃₉ est Ser ou Tyr ; et Z₂ est -OH ou -NH₂ ;
pourvu que pas plus de trois parmi Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ et Xaa₂₈ soient Ala ; et pourvu que, si Xaa₁ est His, Arg ou Tyr, alors au moins un parmi Xaa₃, Xaa₄ et Xaa₉ est Ala ; et les sels pharmaceutiquement acceptables de ceux-ci ; et dans lequel Xaa₁, Xaa₃ ou Xaa₉ est Ala.

2. Composé selon la revendication 1, dans lequel Xaa₂ est Gly.

3. Composé selon la revendication 1 ou 2, dans lequel Xaa₄ est Ala.

4. Composé selon la revendication 2 ou 3, dans lequel Xaa₁₄ est Leu, la pentylglycine ou Met.

5. Composé selon la revendication 4, dans lequel Xaa₂₅ est Trp ou Phe.

6. Composé selon la revendication 5, dans lequel Xaa₆ est Ala, Phe ou la naphtylalanine ; Xaa₂₂ est Phe ou la naphtylalanine ; et Xaa₂₃ est Ile ou Val.

7. Composé selon la revendication 1 ou 6, dans lequel Xaa₃₁, Xaa₃₆, Xaa₃₇ et Xaa₃₈ sont choisis indépendamment dans le groupe constitué par Pro, l'homoproline, la thioproline et une N-alkylalanine.

8. Composé selon la revendication 1 ou 6, dans lequel Xaa₃₉ est Ser.

9. Composé selon l'une quelconque des revendications 1, 7 ou 8, dans lequel Z₂ est -NH₂.

10. Composé selon la revendication 1, qui a une séquence d'acides aminés choisie parmi les SEQ ID n° 72, 73, 75-78, 80-83, 86, 88-90 et 92-93.

11. Composition comprenant un composé selon l'une quelconque des revendications 1 à 10, dans un véhicule pharmaceutiquement acceptable.

12. Utilisation d'un composé selon la revendication 1 ou 10, pour la fabrication d'une composition pharmaceutique pour le traitement du diabetes mellitus.

13. Utilisation selon la revendication 12, ladite composition comprenant en outre une quantité thérapeutiquement efficace d'une insuline.

14. Utilisation d'un composé selon la revendication 1 ou 10, pour la fabrication d'une composition pharmaceutique pour le traitement d'un état hyperglycémique chez un mammifère.

15. Composé peptidique de formule (II) (SEQ ID n° 94), dans lequel ledit composé peptidique présente une activité agoniste de l'exendine : dans lequel
Xaa₁ est His, Arg, Tyr, Ala, Norval, Val, Norleu ou le 4-imidazopropionyle ;
Xaa₂ est Ser, Gly, Ala ou Thr ;
Xaa₃ est Ala, Asp ou Glu ;
Xaa₄ est Ala, Norval, Val, Norleu ou Gly ;
Xaa₅ est Ala ou Thr ;
Xaa₆ est Phe, Tyr ou la naphtylalanine ;
Xaa₇ est Thr ou Ser ;
Xaa₈ est Ala, Ser ou Thr ;
Xaa₉ est Ala, Norval, Val, Norleu, Asp ou Glu ;
Xaa₁₀ est Ala, Leu, Ile, Val, la pentylglycine ou Met ;
Xaa₁₁ est Ala ou Ser ;
Xaa₁₂ est Ala ou Lys ;
Xaa₁₃ est Ala ou Gln ;
Xaa₁₄ est Ala, Leu, Ile, la pentylglycine, Val ou Met ;
Xaa₁₅ est Ala ou Glu ;
Xaa₁₆ est Ala ou Glu ;
Xaa₁₇ est Ala ou Glu ;
Xaa₁₉ est Ala ou Val ;
Xaa₂₀ est Ala ou Arg ;
Xaa₂₁ est Ala ou Leu, ou Lys- NH^{ε}-R, dans lequel R est Lys, Arg, un alcanoyle à chaîne linéaire ou ramifié en C₁ à C₁₀ ou un cycloalkylalcanoyle;
Xaa₂₂ est Phe, Tyr ou la naphtylalanine ;
Xaa₂₃ est Ile, Val, Leu, la pentylglycine, la tert-butylglycine ou Met;
Xaa₂₄ est Ala, Glu ou Asp ;
Xaa₂₅ est Ala, Trp, Phe, Tyr ou la naphtylalanine ;
Xaa₂₆ est Ala ou Leu ;
X₁ est Lys Asn, Asn Lys, Lys- NH^{ε}-R Asn, Asn Lys- NH^{ε}-R, Lys-NH^{ε}-R Ala, Ala Lys- NH^{ε}-R, dans lequel R est Lys, Arg, un alcanoyle à chaîne linéaire ou ramifié en C₁ à C₁₀ ou un cycloalkylalcanoyle ; et dans lesquelles Xaa₃₁, Xaa₃₆, Xaa₃₇ et Xaa₃₈ sont choisis indépendamment dans le groupe constitué par Pro, l'homoproline, 3Hyp, 4Hyp, la thioproline, une N-alkylglycine, une N-alkylpentylglycine et une N-alkylalanine ; Xaa₃₉ est Ser ou Tyr ; et Z₂ est -OH ou -NH₂ ;
pourvu que pas plus de trois parmi Xaa₃, Xaa₄, Xaa₅, Xaa₆, Xaa₈,
Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ et Xaa₂₈ soient Ala ; et pourvu aussi que, si Xaa₁ est His, Arg ou Tyr, alors au moins un parmi Xaa₃, Xaa₄ et Xaa₉ est Ala ; et les sels pharmaceutiquement acceptables de ceux-ci ; et dans lequel Xaa₁, Xaa₃ ou Xaa₉ est Ala.

16. Composé selon la revendication 15, dans lequel Xaa₂ est Gly.

17. Composé selon la revendication 15, dans lequel Xaa₄ est Ala.

18. Composé selon la revendication 15, dans lequel Xaa₁₄ est Leu, la pentylglycine ou Met.

19. Composé selon la revendication 15, dans lequel Xaa₂₅ est Trp ou Phe.

20. Composé selon la revendication 15, dans lequel Xaa₆ est Ala, Phe ou la napthtylalanine ; Xaa₂₂ est Phe ou la naphtylalanine ; et
Xaa₂₃ est Ile ou val.

21. Composé selon la revendication 15, dans lequel Xaa₃₁, Xaa₃₆, Xaa₃₇ et Xaa₃₈ sont choisis indépendamment dans le groupe constitué par Pro, l'homoproline, la thioproline et une N-alkylalanine.

22. Composé selon la revendication 15 ou 21, dans lequel Xaa₃₉ est Ser.

23. Composé selon la revendication 15, dans lequel Z₂ est -NH₂.

24. Composé selon la revendication 15, dans lequel X₁ est Lys Asn, Lys-NH^{ε}-R Asn, ou Lys-NH^{ε}-R Ala, dans lequel R est Lys, Arg, un alcanoyle à chaîne linéaire ou ramifié en C₁ à C₁₀.

25. Composé selon la revendication 15, dans lequel Xaa₂₁ est Lys-NH^{ε}-R dans lequel R est Lys, Arg, un alcanoyle à chaîne linéaire ou ramifié en C₁ à C₁₀ ou un cycloalkyl-alcanoyle.

26. Composé selon la revendication 15, qui a une séquence d'acides aminés choisie parmi les SEQ ID n° 105, 106, 103 et 110.

27. Composition comprenant un composé selon la revendication 15 ou 26, dans un véhicule pharmaceutiquement acceptable.
